(19) Europäisches Patentamt / European Patent Office / Office européen des brevets

(11) **EP 2 772 548 A1**

(12) **EUROPEAN PATENT APPLICATION**

(43) Date of publication:
**03.09.2014 Bulletin 2014/36**

(21) Application number: **13156942.8**

(22) Date of filing: **27.02.2013**

(51) Int Cl.:
*C12Q 1/02* (2006.01)          *C12Q 1/37* (2006.01)
*G01N 33/50* (2006.01)         *G01N 33/542* (2006.01)
*G01N 33/68* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Universität Ulm**
**89081 Ulm (DE)**

(72) Inventors:
• **Johnsson, Nils**
  **89081 Ulm (DE)**
• **Moreno Andrés, Daniel**
  **89073 Ulm (DE)**
• **Dünkler, Alexander**
  **89134 Blaustein (DE)**

(74) Representative: **Lange, Sven**
**Hertin und Partner**
**Rechts- und Patentanwälte**
**Kurfürstendamm 54/55**
**10707 Berlin (DE)**

(54) **A fluorescent reporter for determining molecular interactions**

(57)    The invention relates to a fluorescent reporter for determining molecular interactions, comprising an ubiquitin sub-domain attached to two flanking fluorescent marker molecules, and a system and method for determining molecular interactions, preferably between a protein and another molecule.

Fig. 1

SEQUENCE LISTING

<110>   Universität Ulm

<120>   A fluorescent reporter for determining molecular interactions

<130>   XII 170/13

<160>   2

<170>   PatentIn version 3.3

<210>   1
<211>   36
<212>   PRT
<213>   artificial

<220>
<223>   Nub

<400>   1

```
Met Gln Ile Phe Val Lys Thr Leu Thr Gly Lys Thr Ile Thr Leu Glu
1               5                   10                  15


Val Glu Ser Ser Asp Thr Ile Asp Asn Val Lys Ser Lys Ile Gln Asp
            20                  25                  30


Lys Glu Gly Ile
        35
```

<210>   2
<211>   42
<212>   PRT
<213>   artificial

<220>
<223>   Cub

<400>   2

```
Gly Ile Pro Pro Asp Gln Gln Arg Leu Ile Phe Ala Gly Lys Gln Leu
1               5                   10                  15


Glu Asp Gly Arg Thr Leu Ser Asp Tyr Asn Ile Gln Lys Glu Ser Thr
            20                  25                  30


Leu His Leu Val Leu Arg Leu Arg Gly Gly
        35                  40
```

**Description**

The invention relates to a fluorescent reporter for determining molecular interactions, comprising an ubiquitin (Ub) sub-domain attached to two fluorescent marker molecules, and a system and method for determining molecular interactions, preferably between a protein and another molecule, such as protein-protein interactions.

The invention therefore provides in a preferred embodiment a fluorescent reporter for monitoring protein-protein interactions in living cells. The approach is based on the Split-Ubiquitin method and uses the ratio of two auto-fluorescent reporter proteins as a signal for interaction. In one embodiment, the mating of two haploid yeast cells initiates the analysis and the interactions are followed online by two-channel time-lapse microscopy of the diploid cells during their first cell cycle. Using this approach the invention enables high spatiotemporal resolution to visualize the differences between the interactions of the microtubule binding protein Stu2p with two of its binding partners, monitor the transient association of a Ran-GTPase with its receptors at the nuclear pore and distinguish between protein interactions at the polar cortical domain at different phases of polar growth. These examples further demonstrate that protein-protein interactions identified from large-scale screens can be effectively followed up by high-resolution single cell analysis according to the present invention.

## BACKGROUND OF THE INVENTION

A mechanistic understanding of cellular biology requires a comprehensive knowledge about the protein interactions of the cell (Breitkreutz et al., 2010; Gavin et al., 2002; Krogan et al., 2006; Uetz et al., 2000; Yu et al., 2008). Split-protein-sensors comprise a family of related techniques that contributed in small- and large-scale experiments to this cumulative endeavour (Hruby et al., 2011; Lowder et al., 2011; Miller et al., 2005; Stynen et al., 2012; Tarassov et al., 2008). The Split-Ubiquitin method (SplitUb) is the prototype of these techniques (Müller and Johnsson, 2008; Stynen et al, 2012). Here, the N- and C-terminal halves of Ubiquitin (Nub and Cub) are coupled separately to the two proteins under study. Upon interaction of the fusion proteins, Nub and Cub are forced into close proximity and reassemble into a native-like Ubiquitin (Ub). The native-like Ub is recognized by Ub-specific proteases that cleave off a reporter protein that was genetically attached to the C-terminus of Cub (Johnsson and Varshavsky, 1994). The cleavage of the Split-Ub reporter orothic acid decarboxylase (Ura3p, CRU) from Cub leads to a qualitative difference in bulk cell growth (Wittke et al., 1999). This and other proteome-wide interaction techniques produce binary protein-protein interaction maps. The information encoded in these maps could fundamentally transform our understanding of cellular processes. However, to be effectively used in cell biology these networks would need to acquire a spatial as well as temporal dimension in order to place the interactions into a functional and cellular context (Alexander et al., 2009). The reason for the prevalence of binary interaction maps is mainly technical. Currently, robust and easy-to-use approaches for the characterization of cellular protein interactions in space and time are not available.

Due to their indifference in regard to order, location and timing of interactions, large-scale protein interaction networks are still a relatively untapped resource of information that waits to be fully exploited to better understand the workings of the cell. Computational and experimental approaches are currently used to further refine the structure of protein interaction networks. However, as they typically do not consider the important spatial and temporal aspects their immediate use remains rather limited. The reason for this shortcoming is mainly technical. Methods of high-throughput detection are generally not compatible with in depth cellular investigation. Many in vivo techniques require fine-tuning for each investigated pair and are not compatible with a rapid, parallel investigation of several interaction partners. Other split-protein sensors known in the art lack either a comparable spatial (Split-Luciferase), or spatial and temporal resolution (split-GFP (BiFC), Split- DHFR).

Although split ubiquitin systems have been described in the art (US 2004/0170970 A1, US 5,585,245), ubiquitin-dependent protease-based systems have not previously been employed in combination with fluorescent markers that allow determination of temporal aspects and/or the physical location of molecular interactions. Approaches have been disclosed in the art that utilise single fluorescent markers in combination with split ubiquitin systems (Laser, H. et al., 2000). Such approaches enable only the detection of the presence of a molecular interaction and do not enable determination of temporal or cell-location specific aspects of the interaction. No combination of split ubiquitin with multiple fluorescent markers has been previously attempted.

The present invention therefore provides a new reporter, method and system based on Split-Ub and two spectrally different fluorescent markers (SPLIFF) to monitor the interaction between two molecules with high spatial and temporal resolution. The invention demonstrates that SPLIFF can bridge large-scale protein interaction screens with high-resolution single-cell analysis.

## SUMMARY OF THE INVENTION

In light of the prior art the technical problem underlying the invention was the provision of means for determining molecular interactions, preferably protein-protein interactions, that avoid the disadvantages of those systems known in the art.

This problem is solved by the features of the independent claims. Preferred embodiments of the present invention are provided by the dependent claims.

Therefore, an object of the invention is to provide a fluorescent reporter for determining molecular interactions, comprising a ubiquitin sub-domain attached to two (or more) preferably flanking, fluorescent marker molecules, whereby

- the fluorescent marker molecules exhibit distinct fluorescent properties from one another, such as distinct absorption and/or emission spectra, and

- one of said fluorescent marker molecules is capable of cleavage from the fluorescent reporter via ubiquitin-specific protease activity upon physical interaction between a) said ubiquitin sub-domain of the fluorescent reporter and b) a complementary ubiquitin sub-domain present on a separate molecule.

One important feature of the invention that provides differentiation from the prior art is the use of multiple, preferably two, fluorescent markers in combination with the split ubiquitin system. The use of two or more fluorescent markers enables relative quantification of the intensities and/or locations of the two markers, thereby allowing concrete measurements of location- and/or temporal-specific aspects of the molecular interaction. Approaches in the art utilising single fluorescent markers in combination with split ubiquitin do not provide suitable means for relative quantification and therefore lack the benefits of the reporter as described herein.

Contrary to general expectation, the incorporation of an additional fluorescent marker (for example a fluorescent reporter protein; such as Cherry or any other fluorescent marker protein) between the protein of interest and the Ub sub-domain (preferably Cub) did not result in a general loss of sensitivity to measure (quantify and/or detect) the interaction between the protein of interest and its binding partners.

The introduction of an additional fluorescent marker between the protein of interest and the Ub sub-domain could be assumed, for example, to potentially disrupt an interaction surface of the protein to be investigated or potentially increase the physical distance between the molecular interaction of interest and the formation of the quasi-native ubiquitin moiety. A skilled person may have considered such an amendment to systems known in the art as disadvantageous.

A skilled person could also have assumed that the addition of a fluorescent marker between the protein of interest and the Ub sub-domain would lead to a decreased likelihood of the formation of a quasi-native ubiquitin moiety. For example, if the molecular interaction to be determined was formed preferably (for example due to strong affinity) over formation of the quasi-native ubiquitin moiety, then the additional fluorescent marker between the Cub and protein of interest could, due to steric effects in light of its positioning between the protein and Ub sub-domain, subsequently distort formation of the quasi-native ubiquitin moiety.

Due to the steric considerations in adding an additional fluorescent marker, a skilled person would not have expected that a) the interaction to be determined and b) the formation of the quasi-native ubiquitin moiety, could both be maintained in such a way that the method was not detrimentally affected by a loss of sensitivity. It was a surprising finding that, even in light of potential steric or structural modifications of adding an additional marker within the reporter, the binding properties of the interaction of interest and the quasi-Ub formation (leading to cleavage) are maintained to enable an effective split Ub system. This is supported by all analysed interactions that have been carried out to date. The production itself and the functionality of the reporter as described herein is therefore an unexpected development or improvement of the prior art, which would not have been reasonably expected by a skilled person.

The invention therefore relates to a fluorescent reporter, characterised in that the ubiquitin sub-domain of the fluorescent reporter and the complementary ubiquitin sub-domain are capable of physically interacting with each other to form a quasi-native ubiquitin moiety, recognisable by an ubiquitin-specific protease.

In a preferred embodiment the invention relates to a fluorescent reporter, characterised in that the ubiquitin sub-domain of the fluorescent reporter is a C-terminal sub-domain (Cub) and the complementary ubiquitin sub-domain is an N-terminal sub-domain (Nub). As is demonstrated in the examples, this particular arrangement of C- and N-terminal sub-domains enables determination of molecular interactions in single living cells by time-lapse fluorescence microscopy.

It was surprising, that the split-ubiquitin systems described in the art could be adapted to enable not only reporters based on cell survival read-outs, for example proteins or enzymes responsible for amino acid metabolism in the presence or absence of essential amino acids, but to enable real-time visualisation of molecular interactions using fluorescence marker molecules. The split-ubiquitin systems known in the art have until now not been coupled with multiple fluorescent markers. The present invention therefore represents a surprising and advantageous development of the art, enabling a cell-location specific and temporal determination of protein-protein interactions or interactions between other molecules labelled with the appropriate components of the invention.

In another embodiment of the invention the fluorescent reporter is characterised in that the fluorescent marker molecule capable of cleavage from the fluorescent reporter is either

- degradable after cleavage, leading to a loss of fluorescence of said marker molecule, or

- non-degradable after cleavage, enabling spatial separation of the two fluorescent marker molecules.

Degradable fluorescent markers produce a loss of signal after physical interaction. This approach is particularly suited for two-channel time-lapse fluorescence microscopy. GFP is one example of a degradable marker applied in the invention. Non-degradable markers may be applied when detection techniques are employed that measure spatial dissociation between the two fluorescent markers. Fluorescent markers are known in the art that may be used or modified to be appropriate for non-degradable applications of the method and reporter described herein.

In one embodiment of the invention the fluorescent reporter is characterised in that a protein to be investigated for molecular interaction with another molecule, said other molecule being preferably a protein, nucleic acid, small molecule, antibody, lipid or other chemically or biologically relevant molecule, is covalently attached to the fluorescent reporter, preferably as a fusion protein.

A further aspect of the invention therefore relates to the reporter as described herein in combination with another molecule, which is to be investigated for physical interaction with said first protein of interest, whereby said other molecule is attached to a complementary ubiquitin sub-domain. The other molecule is preferably a protein, nucleic acid, small molecule, antibody, lipid or other chemically or biologically relevant molecule. The "combination" of the reporter and other molecule may relate to any kind of combination, for example in a cell, during determination of a molecular interaction, in a kit, whereby one or more nucleic acid molecules encoding both the reporter and corresponding complementary ubiquitin sub-domain are present, or in vitro, if the interaction is to be determined in an in vitro, for example cell free, environment. The invention therefore relates in a preferred embodiment to a pair of fusion proteins, or one or more nucleic acid molecules encoding such fusion proteins, whereby the pair relates to a reporter as described herein, preferably fused to a first protein of interest, and a corresponding complementary ubiquitin sub-domain fused to a second protein for determination of protein interaction between said first and second proteins.

In one embodiment of the invention the fluorescent reporter is characterised in that the fluorescent marker molecules are fluorescent proteins, such as Cherry or GFP, or fluorophores, whereby said fluorophores are for example attached to proteins or protein domains that are fused either N- and/or C-terminally to the ubiquitin sub-domain of the reporter, for example via a SNAP-Tag, CLIP-Tag or HALO-Tag.

In one embodiment of the invention the fluorescent reporter is characterised in that the ubiquitin sub-domain and flanking fluorescent proteins are covalently attached as a fusion protein. In one embodiment of the invention the fluorescent reporter is characterised in that said fluorescent reporter is covalently attached to the N- and/or C-terminus of a protein to be investigated for physical interaction, preferably as a fusion protein.

Fusion proteins are advantageous for various reasons, particularly due to the ease of construction and implementation, especially in the context of a high throughout screening process, whereby multiple proteins, potentially in form of a library or other screening platform, require labelling with the reporter as described herein. Genetic engineering approaches are known to skilled practitioners, including recombination-based approached or traditional restriction-based cloning, thereby allowing the construction of fusion proteins that comprise the reporter of the ubiquitin sub domain and fluorescent protein markers, in addition to directly labelling the protein of interest as a single fusion protein, comprising the protein to be investigated fused to the reporter at either the N or C-terminus.

In preferred embodiments of the present invention the fluorescent reporter is characterised in that

- said fluorescent reporter comprises of Cherry fluorescent protein, Cub and green fluorescent protein (GFP), in the provided order, whereby a protein to be investigated for physical interaction is covalently attached to Cherry fluorescent protein, preferably as a fusion protein, and GFP is capable of cleavage from the fluorescent reporter via ubiquitin-specific protease activity (upon interaction wit a corresponding complementary Ub sub-domain), or

- said fluorescent reporter comprises of Green fluorescent protein, Cub and of Cherry fluorescent protein, in the provided order, whereby a protein to be investigated for physical interaction is covalently attached C-terminally to Cherry fluorescent protein, preferably as a fusion protein, and GFP-Cub is capable of cleavage from the protein to be investigated for physical interaction via ubiquitin-specific protease activity (upon interaction wit a corresponding complementary Ub sub-domain).

These particular constructs have been shown to be not only functional but also surprisingly amenable to cloning and labelling in vivo or in vitro, enabling recombination-based cloning approaches to introduce these constructs as fusion constructs, attached to any given protein to be assessed for molecular interaction.

A further aspect of the invention relates to a nucleic acid molecule encoding a fluorescent reporter or fusion protein according to any one of the preceding claims. Nucleic acids include preferably DNA and RNA, or other appropriate synthetic nucleic acids, and are preferably provided as expression vectors, capable of replication in various biological systems.

A further aspect of the invention relates to a cell comprising a fluorescent reporter or fusion protein according to any one of the preceding claims or a nucleic acid molecule encoding said reporter or fusion protein according to any one of the preceding claims. The present invention may be provided in prokaryotic or eukaryotic cells and is applicable in mammalian cell culture in addition to the yeast examples shown herein.

A further aspect of the invention relates to a system for determining physical interaction between a protein and another molecule, said other molecule being preferably a protein, nucleic acid, small molecule, antibody, lipid or other chemically or biologically relevant molecule, comprising

- a fluorescent reporter according to any one of claims 1 to 9 attached, preferably covalently, more preferably as a fusion protein, to said first protein to be investigated for physical interaction with another molecule, and

- another molecule to be investigated for physical interaction with said first protein, attached, preferably covalently, to a complementary ubiquitin sub-domain,

whereby

- physical interaction between said first protein and said other molecule enables physical interaction between the ubiquitin sub-domain of the fluorescent reporter and the complementary ubiquitin sub-domain of said other molecule to form a quasi-native ubiquitin moiety, recognisable by an ubiquitin-specific protease.

The invention also relates to a method for determining a physical interaction between a protein and another molecule, said other molecule being preferably a protein, nucleic acid, small molecule, antibody, lipid or other chemically or biologically relevant molecule, comprising

- providing a fluorescent reporter according to any one of claims 1 to 9 attached, preferably as a fusion protein, to said first protein to be investigated for physical interaction with another molecule, and

- providing another molecule to be investigated for physical interaction with said first protein, attached, preferably as a fusion protein, to a complementary ubiquitin sub-domain,

whereby

- when physical interaction between said first protein and said other molecule occurs, preferably in a cell, physical interaction between the ubiquitin sub-domain of the fluorescent reporter and the complementary ubiquitin sub-domain of said other molecule is enabled to form a quasi-native ubiquitin moiety, recognisable by an ubiquitin-specific protease, and

- ubiquitin-specific protease activity leads to cleavage and subsequent degradation of the cleavable fluorescent marker molecule leading to a loss of fluorescence of said marker, or ubiquitin-specific protease activity leads to cleavage and subsequent spatial separation of the two fluorescent markers.

In one embodiment the method of the invention is used for determining the temporal and/or spatial distribution of a physical interaction between a protein and another molecule, said other molecule being preferably a protein, nucleic acid, small molecule, antibody, lipid or other chemically or biologically relevant molecule, using a method according to the preceding claim, comprising

- the use of fluorescent imaging and/or detection means, preferably two-channel time lapse fluorescent microscopy, fluorescence energy resonance transfer (FRET) or fluorescence cross correlation spectroscopy, to determine changes in fluorescence of the cleavable and/or non-cleavable fluorescent marker molecules over time and/or with regard to the spatial distribution of fluorescent signals,

whereby

- a change in the ratio of fluorescence intensities of the cleavable and non-cleavable fluorescent marker molecules, preferably a reduction in fluorescence intensity of the cleavable fluorescent marker molecule in relation to the non-cleavable fluorescent marker molecule, indicates physical interaction between a first protein and another molecule, and/or

- a change in relative distance between the cleavable and non-cleavable fluorescent marker molecules indicates physical interaction between a first proteins and another molecule, whereby the extent of co-localization or energy-transfer of the two fluorescent reporter proteins indicates the amount of cleaved fluorescent marker and thus the degree of physical interaction.

A further aspect of the invention relates to a kit comprising components suitable for carrying out the method as described herein, comprising at least a fluorescent reporter or fusion protein as described herein, a nucleic acid molecule encoding a fluorescent reporter or fusion protein as described herein, and/or a cell comprising a fluorescent reporter or fusion protein or nucleic acid molecule as described herein. A kit is envisaged comprising a nucleic acid molecule comprising the reporter as described herein. Such a nucleic acid molecule is easily used as a template for cloning or amplification of the reporter, in order to introduce the reporter construct into any given cell or cell line in the context of creating an appropriately labelled protein to be investigated.

The invention therefore enables a novel technique, SPLIFF, which is based on the Split-Ubiquitin method and uses the ratio of two fluorescent reporter proteins as signal for interaction. The interactions can be recorded in single living cells by time-lapse fluorescence microscopy. To demonstrate the general applicability and robustness of the novel method, known and novel protein interactions were analyzed in the nucleus, at the nuclear pore, in the cytosol, at the polar cortical domain and of the microtubule cytoskeleton of yeast cells with unprecedented temporal and spatial resolution. The method allows tracing protein interactions throughout the cell cycle, to detect spatial gradients of protein interactions, to differentiate between interactions of one protein with two different ligands, and to give a quantitative determination for the stability of a certain protein complex.

The invention therefore relates to a method, system or kit as described herein incorporating the use of multiple complementary Ub sub-domains, which may be of the same or different amino acid sequence and structure, each coupled with distinct fluorescent markers and each coupled to different molecules to be assessed for physical interaction with any given first protein that is attached to the reporter as described herein. Such an approach enables the determination of multiple molecular interactions with any given first protein of interest simultaneously due to the different optical and/or fluorescent characteristics of the different fluorescent markers applied. This approach may only be limited by the number of channels for detection on the fluorescence detection device.

The invention provides a significant technical advance in analyzing protein-protein interactions in single cells. The wide applicability of SPLIFF should allow to bridge large-scale protein interaction screens with high-resolution single cell analysis. The invention provides examples from three newly performed interaction screens. Selected hits were subsequently investigated by SPLIFF. The method requires only standard instrumentation. The analysis and interpretation of the data require only tools that are already widespread in the community through their common use in fluorescence microscopy. All these features increase the potential of SPLIFF to be widely accepted by cell biologists to gain deeper insights into molecular interactions, such as protein interactions and protein interaction networks.

For example, in yeast, protein and genetic interaction networks are now available on a genome wide scale. Combining the information obtained from both networks promises to predict the functions and the workings of small networks and their protein members. However, this is true for only a minority of the investigated networks as the nature of the data and their presentation leaves too many possible solutions. To obtain additional constraints for the analysis of small networks the interactions among a subset of all yeast proteins can be measured. From these raw interaction data constraint interaction networks (CIN) can be derived by at least two types of follow-up experiments: 1. Protein interaction measurements in certain gene deletion mutants and 2. Interaction measurements of fragments of those proteins that bind multiple ligands. The CINs define interaction states that ideally summarize all interactions of a protein that can occur at the same time. Furthermore, different interaction states are therefore either simultaneously realized in different cellular locales or alternatively represent temporal states in sequels of ordered transformations. Co-localization studies, for example using the reporter of the present invention, of pairs of binding partners introduce further constraints helping to distinguish between these alternatives. Additional constraints for the functional interpretation of network members are obtained by measuring the genetic interaction between genes of binding partners and between alleles of these genes specifically lacking the sequences for the interaction sites.

## DETAILED DESCRIPTION OF THE INVENTION

A "fluorescent reporter" for molecular interaction is to be understood as a molecule, preferably a protein molecule, which is detectable using any method or device based on the detection of fluorescence. The reporter is preferably capable of physical change upon the desired molecular interaction, whereby said physical change is detectable either directly or indirectly. The invention therefore also relates to a protein, or fusion protein, that is a fluorescent reporter.

The "molecular interactions" of the present invention relate to any physical interaction between any given molecular entities. Preferably, a molecule may be coupled to either the reporter of the invention or the corresponding complementary ubiquitin-sub-domain, whereby sufficient physical proximity between the reporter and complementary ubiquitin-sub-domain leads to cleavage and a detectable physical change in the reporter. Preferred molecular interactions relate to protein-protein interactions, which can be assessed using proteins attached to said reporter and said complementary ubiquitin-sub-domain, preferably as fusion proteins.

The term "physical interaction" as used herein relates to any interaction or association between molecules that involves molecular contact. Interactions encompassed by the term physical interaction include those that are of transient or stable nature, including interactions that may potentially lead to chemical reaction and covalent coupling. The preferred physical interactions of the present invention relate to examples such as protein-protein interactions, as are found in protein complexes, or for example antibody-epitope interactions. It is known in the art that proteins often form multi-protein complexes, which represent relatively stable molecular interactions under physiological conditions.

The term "covalent" attachment as used herein refers to a covalent bond as commonly understood by a skilled person. Although covalent attachments are preferred, analogous physical interactions that are not necessarily covalent but functionally equivalent (for example interactions with high specificity and affinity) may also be applied in place of a covalent bond.

Ubiquitin (Ub) is a 76-residue, single-domain protein whose covalent coupling to other proteins yields branched Ub-protein conjugates and plays a role in a number of cellular processes, primarily through routes that involve protein degradation. Unlike the branched Ub conjugates, which are formed posttranslationally, linear Ub adducts are the translational products of natural or engineered Ub fusions. It has been shown that, in eukaryotes, newly formed Ub fusions are rapidly cleaved at the Ub-polypeptide junction by Ub-specific proteases (UBPs). In the yeast *Saccharomyces cerevisiae,* there are at least five species of UBP. Recent work has shown that the cleavage of a Ub fusion by UBPs requires the folded conformation of Ub, because little or no cleavage is observed with fusions whose Ub moiety was conformationally destabilized by single-residue replacements or a deletion distant from the site of cleavage by UBPs.

The present invention is based on previous work relating to the split-ubiquitin system, whereby a fusion protein comprising a ubiquitin sub-domain is useful, for example, in a method for studying the molecular interactions between members of a specific-binding pair. A "specific binding pair", as used herein, refers to a pair of molecules, which bind specifically to one another when incubated under physiological conditions. Typically, although not necessarily, both members of the specific binding pair are proteins and/or peptides. It is also possible that the reporter system of the present invention is applied for binding or interaction investigation between a protein and another molecule. Essentially any two biologically relevant molecules may be coupled to either the reporter or the complementary ubiquitin sub-domain for determination of their interaction. For convenience, the term "protein(s)" will be used throughout the description of the present invention in the context of studying protein and/or peptide interaction as a shorthand expression for "protein(s) or peptide(s)". The two members of a specific-binding pair may also be referred to as "ligand" or "affinity reagent", for example, in the case of "antigen" and "antibody", respectively.

Briefly, it has been demonstrated that an N-terminal ubiquitin sub-domain and a C-terminal ubiquitin sub-domain, when co-expressed in the same cell as distinct entities, have the ability to associate, reconstituting a ubiquitin molecule which is recognized, and cleaved, by ubiquitin-specific processing proteases which are present in all eukaryotic cells. This reconstituted ubiquitin molecule, which is recognized by ubiquitin-specific proteases, is referred to herein as a "quasi-native" ubiquitin moiety. As disclosed herein, ubiquitin-specific proteases recognize the folded conformation of ubiquitin. Remarkably, ubiquitin-specific proteases retained their cleavage activity and specificity of recognition of the ubiquitin moiety that had been reconstituted from two unlinked ubiquitin sub-domains. The ubiquitin protein has been studied extensively and the DNA sequence encoding ubiquitin has been published (Ozkaynak et al., EMBO J. 6: 1429 (1987)).

The N-terminal sub-domain, as referred to herein, is preferably that portion of the native ubiquitin molecule which folds into the only alpha-helix of ubiquitin interacting with two beta-strands. Generally speaking, this sub-domain comprises amino acid residues from about residue number 1 to about residue number 36. The C-terminal sub-domain of ubiquitin, as referred to herein, is preferably that portion of the ubiquitin which is not a portion of the N-terminal sub-domain defined in the preceding paragraph. Generally speaking, this sub-domain comprises amino acid residues from about 37 to about 76. It should be recognized that by using only routine experimentation it is possible to define with precision the minimum requirements at both ends of the N-terminal sub-domain and the C-terminal sub-domain which are necessary to be useful in connection with the present invention.

Preferred Nub and Cub sequences are as below:

Nub (SEQ ID No. 1):MQIFVKTLTGKTITLEVESSDTIDNVKSKIQDKEGI

Cub (SEQ ID NO. 2): GIPPDQQRLIFAGKQLEDGRTLSDYNIQKESTLHLVLRLRGG

Variations in amino acid or nucleic acid sequence to the Nub or Cub as described herein, that lead to functionally analogous sequences, are additionally encompassed in the scope of the invention. For example, protein or nucleic acid sequences of more than 80%, preferably 90%, more preferably 95% sequence identity with analogous function are encompassed in the invention. Analogous function in the context of the present invention relates to maintained split Ub function, whereby Ub sub-domains are capable of interacting thereby forming a quasi-native Ub moiety recognised by Ub-specific proteases. Sequence variants of the claimed nucleic acids, proteins and antibodies, for example defined by the claimed % sequence identity, that maintain the said properties of the invention are also included in the scope of the invention. Sequence identity relates to the percentage of identical nucleotides or amino acids when carrying out a sequence alignment.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein modifications, which occur through substitutions, are also included within the scope of the invention. Substitutions as defined herein are modifications made to the amino acid sequence of the protein, producing a protein which contains a different amino acid sequence than the primary protein without significantly altering the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citrulline and methionine sulfoxide which are neutral non-polar analogs, phenylglycine which is an aromatic neutral analog; cysteic acid which is a negatively charged analog and ornithine which is a positively charged amino acid analog. Like the naturally occurring amino acids, this list is not exhaustive, but merely exemplary of the substitutions that are well known in the art.

One aspect of the invention is applicable to the identification of interacting protein or peptide pairs when one member of the specifically binding pair is known. In a second aspect, the invention relates to compositions and methods for studying the interaction of two members of a specific binding pair, both of which are predetermined. For example, the second aspect of the invention relates to the determination of a predetermined ligand in a sample. The second aspect of the invention is also applicable to the identification of an inhibitor of binding of an analyte (or, more generally, a ligand) to an affinity reagent. The second aspect is also based on the use of ubiquitin fusion constructs wherein N- and C-terminal sub-domains of ubiquitin are fused to first and second members of a specific binding pair, respectively. The specific binding of the members of the specific binding pair is detected by the activation of reporter following cleavage of the C-terminal ubiquitin fusion construct by a ubiquitin-specific protease. In practice, one member of the specific binding pair is selected to mimic the binding characteristics of a ligand to be determined. In the absence of a competitor molecule (e.g., a ligand) in the sample being analyzed, a baseline level of reporter activity is generated based on the binding interaction of the two members of the specific binding pair, which results in cleavage by a ubiquitin-specific protease and "activation", namely cleavage, of the reporter. However, the presence of ligand in the sample being analyzed acts as a competitive inhibitor of the binding between the two ubiquitin fusion constructs, thereby reducing the rate of ubiquitin-specific protease activity and, as a consequence, levels of reporter activity.

The phrase "flanking" as used herein represents the physical binding, preferably covalent binding, more preferably as a fusion protein, between the ubiquitin sub-domain and two fluorescent marker molecules, situated on each side of the ubiquitin sub-domain. The fluorescent markers must not necessarily be diametrically opposed in relation to one another when assessing the three dimensional structure of the reporter. Rather the ubiquitin sub-domain should be positioned appropriately to enable cleavage of one marker upon Ub reconstitution between the Ub sub-domains. The term "flanking" in terms of the fusion proteins includes constructs in which an additional moiety is positioned between the ubiquitin sub-domain and the flanking markers. The fluorescent markers must not necessarily be directly adjacent to the ubiquitin sub-domain positioned between them, although this is a preferred embodiment.

The "fluorescent marker molecules" of the invention relate to any molecule capable of detection using a fluorescent method or device. Examples relate to fluorescent proteins or fluorophores. Fluorescence is commonly understood as the emission of a lower energy (more red-shifted) photon from a molecule (a fluorophore) that is in a singlet excited state due to prior absorption of a higher energy photon (excitation). The singlet excited state is short-lived (less than 100 ns) so fluorescence is observed, effectively, only during excitation. Emission from the long-lived (greater than milliseconds) triplet excited state is known as phosphorescence and can be observed long after the illumination has ceased. For bioluminescence (i.e. enzymatically catalyzed chemiluminescence), the excited state is first produced by a chemical reaction and not by absorption of a photon. Phosphorescent or bioluminescent marker molecules are also encompassed in the present invention.

The term "fluorescent protein" also refers to proteins that are structural or functional homologs of green fluorescent protein and that are able to form an internal visible wavelength fluorophore from their own polypeptide sequence. However, it is important to note that any protein that has a tryptophan, tyrosine, or phenylalanine residue within its sequence is, strictly speaking, a "fluorescent protein" due to the inherent but non-visible fluorescence of these amino acids. A number of other proteins, such as the phycobiliproteins, are visibly "fluorescent proteins" due to the presence of non-proteinaceous chromophores that are associated with the protein.

The term "fluorophore" or "fluorescent marker", as used herein, may refer to a compound, chemical group, or composition that is inherently fluorescent. Fluorophores may contain substituents that alter the solubility, spectral properties or physical properties of the fluorophore. Numerous fluorophores are known to those skilled in the art and include, but are not limited to coumarin, cyanine, benzofuran, a quinoline, a quinazolinone, an indole, a furan, a benzazole, a borapolyazaindacene and xanthenes including fluoroscein, rhodamine and rhodol as well as other fluorophores.

Preferred fluorescent proteins are presented below (Piston, Patterson, Lippincott-Schwartz, Claxton and Davidson, Nikon microscopy resource):

| Protein (Acronym) | Excitation Maximum (nm) | Emission Maximum (nm) | Relative Brightness (% of EGFP) |
|---|---|---|---|
| GFP (wt) | 395/475 | 509 | 48 |
| EGFP | 484 | 507 | 100 |
| Emerald | 487 | 509 | 116 |
| Superfolder GFP | 485 | 510 | 160 |
| Azami Green | 492 | 505 | 121 |
| mWasabi | 493 | 509 | 167 |
| TagGFP | 482 | 505 | 110 |
| TurboGFP | 482 | 502 | 102 |
| AcGFP | 480 | 505 | 82 |
| ZsGreen | 493 | 505 | 117 |
| T-Sapphire | 399 | 511 | 79 |
| EBFP | 383 | 445 | 27 |
| EBFP2 | 383 | 448 | 53 |
| Azurite | 384 | 450 | 43 |
| mTagBFP | 399 | 456 | 98 |
| ECFP | 439 | 476 | 39 |
| mECFP | 433 | 475 | 39 |
| Cerulean | 433 | 475 | 79 |
| mTurquoise | 434 | 474 | 75 |
| CyPet | 435 | 477 | 53 |
| AmCyan1 | 458 | 489 | 31 |
| Midori-Ishi Cyan | 472 | 495 | 73 |
| TagCFP | 458 | 480 | 63 |
| mTFP1 (Teal) | 462 | 492 | 162 |
| EYFP | 514 | 527 | 151 |
| Topaz | 514 | 527 | 169 |
| Venus | 515 | 528 | 156 |
| mCitrine | 516 | 529 | 174 |
| YPet | 517 | 530 | 238 |
| TagYFP | 508 | 524 | 118 |
| PhiYFP | 525 | 537 | 144 |
| ZsYellow1 | 529 | 539 | 25 |
| mBanana | 540 | 553 | 13 |

(continued)

| Protein (Acronym) | Excitation Maximum (nm) | Emission Maximum (nm) | Relative Brightness (% of EGFP) |
|---|---|---|---|
| Kusabira Orange | 548 | 559 | 92 |
| Kusabira Orange2 | 551 | 565 | 118 |
| mOrange | 548 | 562 | 146 |
| mOrange2 | 549 | 565 | 104 |
| dTomato | 554 | 581 | 142 |
| dTomato-Tandem | 554 | 581 | 283 |
| TagRFP | 555 | 584 | 142 |
| TagRFP-T | 555 | 584 | 99 |
| DsRed | 558 | 583 | 176 |
| DsRed2 | 563 | 582 | 72 |
| DsRed-Express (T1) | 555 | 584 | 58 |
| DsRed-Monomer | 556 | 586 | 10 |
| mTangerine | 568 | 585 | 34 |
| mRuby | 558 | 605 | 117 |
| mApple | 568 | 592 | 109 |
| mStrawberry | 574 | 596 | 78 |
| AsRed2 | 576 | 592 | 8 |
| mRFP1 | 584 | 607 | 37 |
| JRed | 584 | 610 | 26 |
| mCherry | 587 | 610 | 47 |
| HcRed1 | 588 | 618 | 1 |
| mRaspberry | 598 | 625 | 38 |
| dKeima-Tandem | 440 | 620 | 21 |
| HcRed-Tandem | 590 | 637 | 19 |
| mPlum | 590 | 649 | 12 |
| AQ143 | 595 | 655 | 11 |

The terms "absorption spectra" and "emission spectra" relate to their common meanings as known in the art. The distinct fluorescent properties of the fluorescent markers of the invention may relate to excitation properties, such as specific excitation and/or emission wavelengths, for example those shown in the table above.

The SNAP-tag or HALO-tag, or other suitable labelling system, may be applied as one or both parts of a fluorescent marker of the reporter as described herein. The HALO- and SNAP-tags may be combined. Alternatively, the CLIP-tag may also be utilised, for example in combination with the SNAP-tag. The CLIP-tag is a mutated version of the SNAP-tag. The CLIP-tag can be orthogonally marked with other suitable derivates. The SNAP-tag protein labelling system enables a specific, covalent attachment of virtually any molecule to a protein of interest. The SNAP-tag is a protein based on human 06-alkylguanine-DNA alkyltransferase (hAGT). SNAP-tag substrates are fluorophores, biotin or beads conjugated to guanine or chloropyrimidine leaving groups via a benzyl linker. In the labelling reaction, the substituted benzyl group of the substrate is covalently attached to the SNAP-tag. SNAP-tag can therefore be applied in the present invention for in vivo or in vitro labelling with various SNAP-tag fluorescent substrates. The HALO-tag protein labelling system is a modified haloalkane dehalogenase designed to covalently bind to synthetic ligands (HaloTag ligands). The synthetic ligands comprise a chloroalkane linker attached to a variety of useful molecules, such as fluorescent dyes, affinity handles, or solid surfaces.

In those embodiments not involving exclusively fusion proteins, the present invention may involve chemical coupling or immobilization between the reporter and the protein to be investigated for interaction, and/or between the other molecule to be investigated for interaction, which is then coupled to the complementary ubiquitin sub-domain.

Such approaches may be suitable for chemical or peptide libraries which are to be labelled, in order to interrogate binding partners for any given protein molecule. The following coupling alternatives are possible, but do not represent limiting embodiments. A common functional target for immobilizing or coupling protein molecules is the amine group (-NH2): This group exists at the N-terminus of each polypeptide chain (called the alpha-amine) and in the side chain of lysine (Lys, K) residues (called the epsilon-amine). Because of its positive charge at physiologic conditions, primary amines are usually outward-facing (i.e, on the outer surface) of proteins; thus, they are usually accessible for conjugation without denaturing the protein structure. Alternatively, coupling through Sulfhydryl Groups may be employed, as often it is advantageous to immobilize or couple affinity ligands through functional groups other than just amines. In particular, the thiol group can be used to direct coupling reactions away from active centres or binding sites on certain protein molecules. Furthermore, Maleimide-activated chemistry could be applied. Maleimide-activated reagents react specifically with sulfhydryl groups (-SH) at near neutral conditions (pH 6.5-7.5) to form stable thioether linkages. Coupling through Carbonyl (Sugar) Groups is a further possibility. Hydrazide-activated resins and compounds will conjugate with carbonyls of oxidized carbohydrates (sugars) at pH 5 to 7, resulting in formation of hydrazone bonds. Coupling through Carboxyl Groups may also be used. The carboxyl group is a frequent constituent of many biological molecules. Peptides and proteins contain carboxyls (-COOH) at the C-terminus of each polypeptide chain and in the side chains of aspartic acid (Asp, D) and glutamic acid (Glu, E). Like primary amines, carboxyls are usually on the surface of protein structure.

The phrase "degradable after cleavage" refers to the capability of a molecular marker to be degraded by either exogenous or endogenous degradation mechanisms, preferably protease activity in the case of fluorescent proteins under physiological conditions. "Non-degradable" refers to markers that are either not quickly or not easily degraded in comparison to the degradable markers, whereby fluorescence can be detected after cleavage. Most fluorescent molecules are capable of degradation or reduction in signal over longer time spans. The phrase non-degradable therefore refers to a relatively stable or less-degradable nature in comparison to the degradable markers of the invention.

A "protein to be investigated for molecular interaction with another molecule" relates to any given protein or peptide for which the presence or absence of a specific physical interaction between a protein and another molecule is to be tested. Said "other molecule" is preferably a protein, thereby enabling protein-protein interaction studies but may be any ligand, affinity reagent or other molecule, which is capable of interacting with a protein. Examples of molecules known to interact with proteins are known to skilled practitioners and include but are not limited to a nucleic acid, small molecule, antibody, lipid or other chemically or biologically relevant molecule.

The quasi-native ubiquitin reconstitution according to the invention for determining interaction between members of a specific-binding pair can be carried out in a number of formats. Common to such formats is the use of two DNA-based expression constructs. DNA-based expression constructs are genetic elements, which can express the desired proteins within the experimental system employed. The starting material for such expression constructs will typically be a well-characterized expression vector (e.g., a plasmid), which contains regulatory elements (e.g., the origin of replication, promoters, etc.) which are suitable for use within a given experimental system. Many expression vectors suitable for use in a variety of experimental systems have been reported and are used routinely by those skilled in the art. Alternatively, the reporter construct and corresponding complementary Ub construct may be integrated into the genome of the given cell in which the interaction is to be detected. The endogenous promoters of the genes corresponding to the proteins to be investigated may therefore be used to drive expression of the protein "tagged" with the reporter of the invention and/or the corresponding protein to be assessed for interaction that is "tagged" with a construct encoding the corresponding complementary Ub sub-domain.

An alternative to co-expression of the two DNA-based expression constructs is the expression of the two vectors in different host cell cultures. Extracts from the two cell cultures (or purified, or partially purified preparations of for example the two fusion proteins) are then combined in presence of a ubiquitin-specific protease. This method permits in vitro analysis of protein interactions. For example, the fusion proteins can be isolated individually, and one can be attached to a solid support (e.g., to a well in a multiwell plate). The second fusion protein is then contacted with the plate-affixed fusion protein in the presence of a ubiquitinspecific protease and, if the proteins of interest, or molecules of interest, bind to one another, the cleavable fluorescent marker will be cleaved from the ubiquitin sub-domain of the reporter. Whether cleavage has occurred can be determined, for example, by the methods described herein.

The term "determine" or "determination", as used in this context, is meant to include qualitative as well as quantitative assessment of binding interactions.

Screening methods are also envisaged, in which libraries of cell lines, each separately (or in combination) are modified to express proteins of interest with the corresponding reporters or complementary constructs. The present invention provides methods to determine whether two proteins bind to each other. When trying to use such methods for the identification of a previously unknown binding partner for a given polypeptide, one preferably will use a library of polypeptides and screen for members of such library that are capable of interacting with the given polypeptide. This is, for example, carried out by constructing a cDNA or genomic library, cloning this library into a vector comprising the reporter construct, and expressing the library of vectors so created in a host cell expressing a fusion protein comprising the given polypeptide and the corresponding complementary Ub construct. This also may be carried out in reverse.

Methods to generate libraries for use in such methods, and how these libraries may be employed to characterize a novel polypeptide interacting with the given polypeptide, are known to a skilled person.

A "cell" as used herein relates to any biological cell. Prokaryotic or eukaryotic cells are encompassed in the invention. Although yeast cells are employed in the examples of the invention, higher eukaryotes are compatible with the present system. Therefore, mammalian cell culture is also envisaged, in which protein interactions, or protein interaction networks, can also be interrogated.

Fluorescent microscopy is known to one skilled in the art. Typically, a sample is illuminated with light of a specific wavelength (or wavelengths) which is absorbed by the fluorophores, causing them to emit light of longer wavelengths (i.e., of a different colour than the absorbed light). The illumination light is separated from the much weaker emitted fluorescence through the use of a spectral emission filter. Typical components of a fluorescence microscope are a light source (such as LED, xenon arc lamp or mercury-vapor lamp), the excitation filter, the dichroic mirror (or dichroic beamsplitter), and the emission filter. The filters and the dichroic are chosen to match the spectral excitation and emission characteristics of the fluorophore used to label the specimen. In this manner, the distribution of a single fluorophore (colour) is imaged at a time. Multi-colour images of several types of fluorophores must be composed by combining several single-colour images.

Most fluorescence microscopes in use are epifluorescence microscopes (i.e., excitation and observation of the fluorescence are from above (epi-) the specimen). These microscopes have become an important tool in the field of biology, enabling more advanced designs, such as the confocal microscope and the total internal reflection fluorescence microscope (TIRF).

Förster (Fluorescence) resonance energy transfer (FRET), resonance energy transfer (RET) or electronic energy transfer (EET) may be applied in the present invention. FRET relates to a mechanism describing energy transfer between two chromophores. In principle, a donor chromophore, initially in its electronic excited state, may transfer energy to an acceptor chromophore through non-radiative dipole-dipole coupling. The efficiency of this energy transfer is inversely proportional to the sixth power of the distance between donor and acceptor making FRET extremely sensitive to small distances. Measurements of FRET efficiency can be used to determine if two fluorophores are within a certain distance of each other. Such measurements are commonly used as a research tool in fields including biology and chemistry and are known to a skilled person.

Fluorescence cross-correlation spectroscopy (FCCS) is similarly applicable for the present invention, providing a method for sensitive measurements distinguishing the labelled molecules. It extends the fluorescence correlation spectroscopy (FCS) procedure by introducing high sensitivity for distinguishing fluorescent particles, which have a similar diffusion coefficient. FCCS uses two species, which are independently labelled with two spectrally separated fluorescent probes. These fluorescent probes are excited and detected by two different laser light sources and detectors commonly known as green and red respectively. FCCS is known to a skilled person and can be applied in the measurements involved in determining and/or identifying the reporter molecule as described herein.

## FIGURES

The figures provided herein represent examples of particular embodiments of the invention and are not intended to limit the scope of the invention. The figures are to be considered as providing a further description of possible and potentially preferred embodiments that enhance the technical support of one or more non-limiting embodiments.

Brief description of the figures:

**Figure 1:** Experimental design of SPLIFF

**Figure 2:** SPLIFF analysis of the Net1 p/Sir2p interaction

**Figure 3:** Comparison between Nub-Cdc14p-induced Net1CCG conversion and GFP-Cdc14p accumulation

**Figure 4:** Protein interactions at the nuclear pore

**Figure 5:** SPLIFF analysis of the interactions between components of the polar cortical domain

**Figure 6:** Map of the cellular location of the Spa2p/Kel1 p interaction

**Figure 7:** Dynamic map of the Nap1 p/Kcc4p interaction

**Figure 8:** Dynamic map of the interactions of Stu2p

Detailed description of the figures:

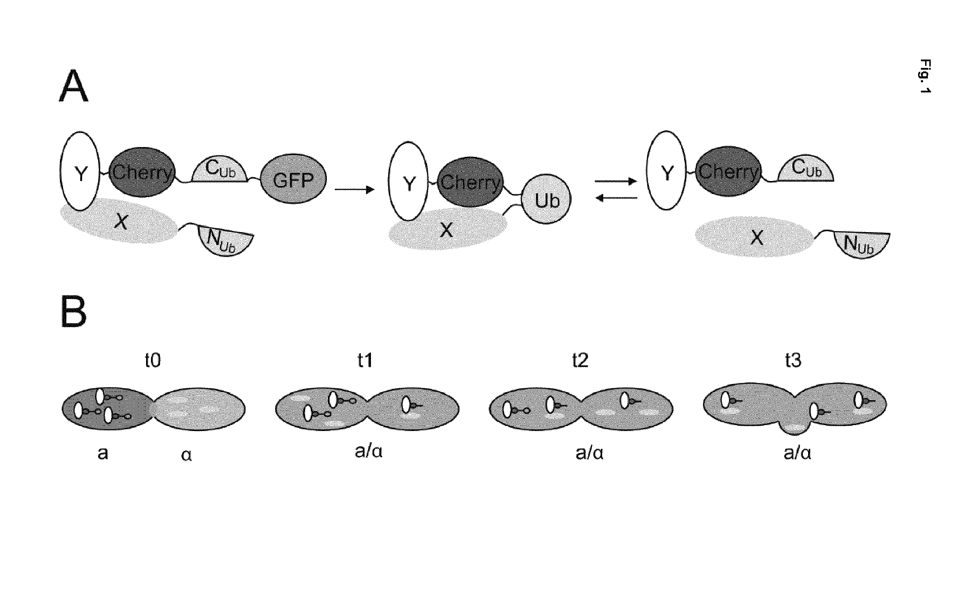

**Figure 1:** Experimental design of SPLIFF. (A) Protein Y coupled to the Cherry-Cub-GFP (Y-CCG) interacts with the Nub-fusion of protein X (Nub-X). Upon reassociation of Nub and Cub the GFP is cleaved off and Y-CCG is converted to Y-CC. The N-terminally exposed arginine leads to rapid degradation of GFP (B) Two yeast cells of the a- and α-mating type expressing Y-CCG and Nub-X respectively fuse at t0. The cytosols mix, Y-CCG and Nub-X interact, leading to the progressive conversion of Y-CCG to Y-CC at t1, t2 and t3.

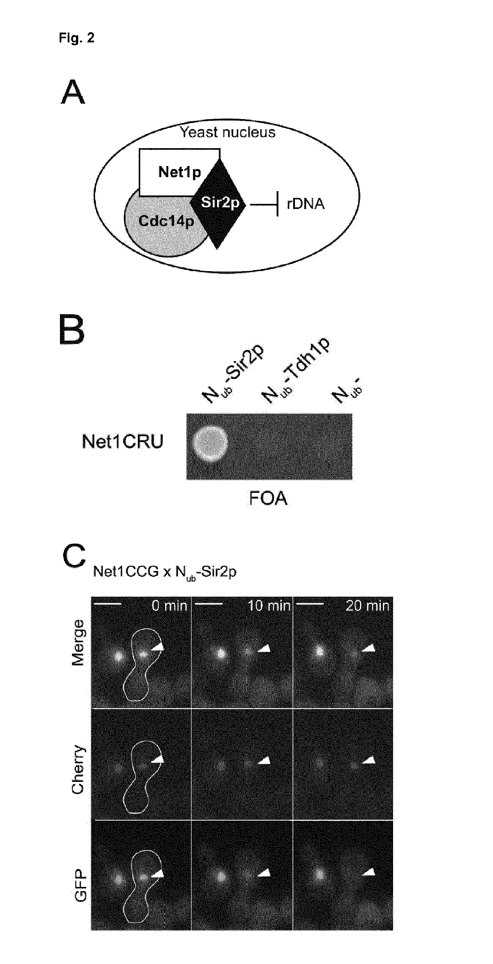

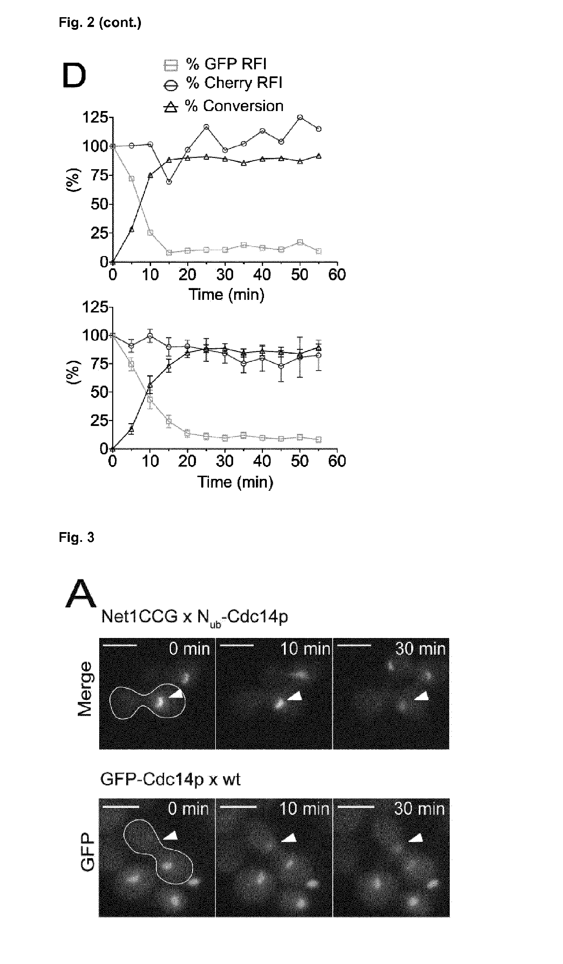

**Figure 2:** SPLIFF analysis of the Net1 p/Sir2p interaction (A) Cartoon of the RENT-complex. (B) Interaction between Net1 p and Sir2p as measured by the Split-Ub growth assay. Cells expressing an interacting $N_u$b-fusion grow on medium containing 5-FOA. (C) Interaction between Net1 p and Sir2p as measured by SPLIFF. Shown are selected frames of the time-lapse analysis of the mating of two yeast cells expressing Net1CCG and Nub-Sir2 respectively (white border). Nuclei of unfarmed cells belong to haploid a-cells. Note the selective loss of green (GFP) fluorescence from the a-cell originated nucleus in the diploid at 10 and 20 min. Time 0 indicates the time point shortly before cell fusion. Scale bar, 5μm. (D) Quantitative analysis of the experiment shown in (C) (upper panel) or the averages of 10 independent experiments (lower panel). The relative fluorescence of GFP (squares), Cherry (circles) and the calculated conversion of Net1CCG to Net1CC (triangles) are plotted against the time (error bars, standard error (s.e.).

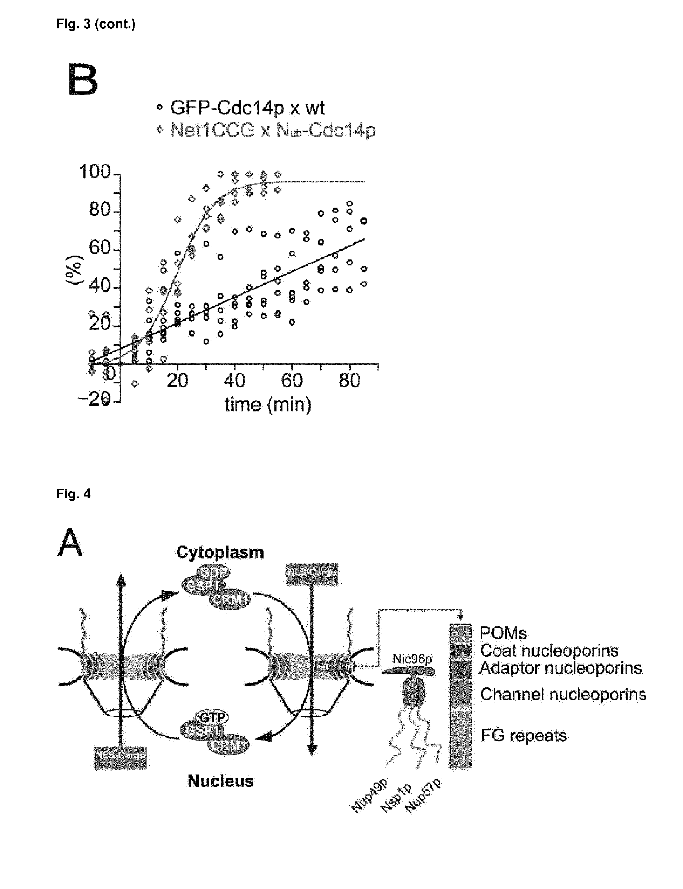

**Figure 3:** Comparison between Nub-Cdc14p-induced Net1CCG conversion and GFP-Cdc14p accumulation. (A) α-cells expressing Nub-Cdc14p were mated with α-cells expressing Net1CCG and the conversion to Net1CC was recorded after mating (upper panel). Lower panel: α-cells expressing GFP-Cdc14p were mated with wild type α-cells and the accumulation of the GFP signal was recorded in the α-cell derived nucleus (white arrowhead) as fraction of total fluorescence measured shortly before cell fusion (t0). The white-framed cells indicate the diploid cells. (B) Pairwise comparison of best fits of independent experiments as shown in (A). Blue curve corresponds to conversion of Net1CCG and black curve to the accumulation of GFP-Cdc14.

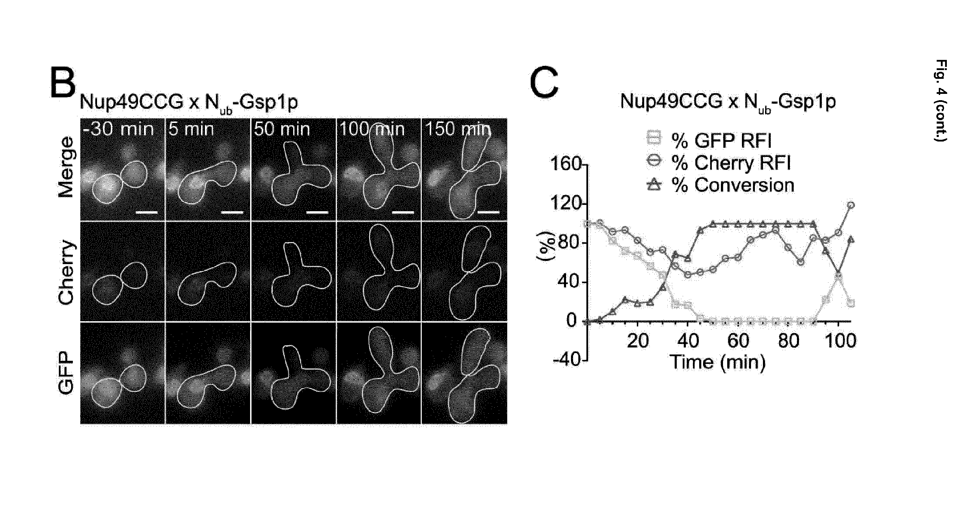

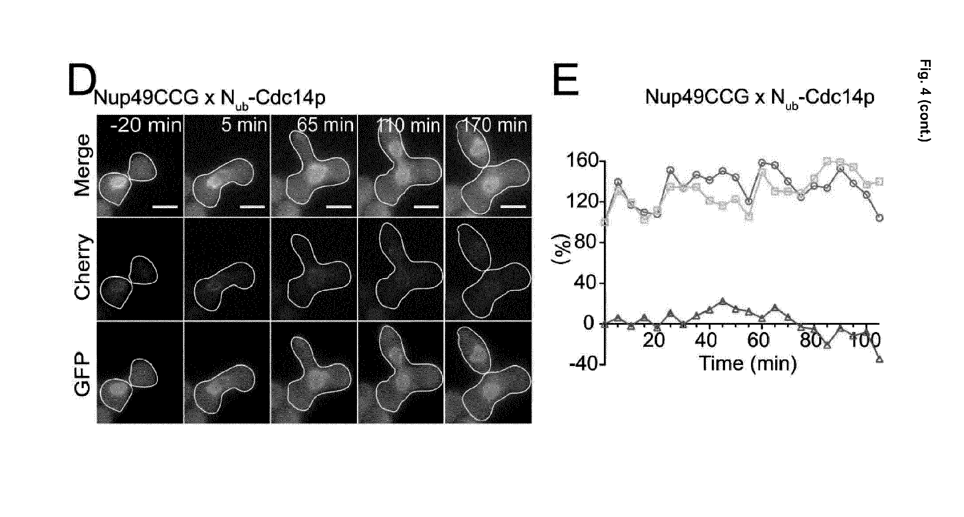

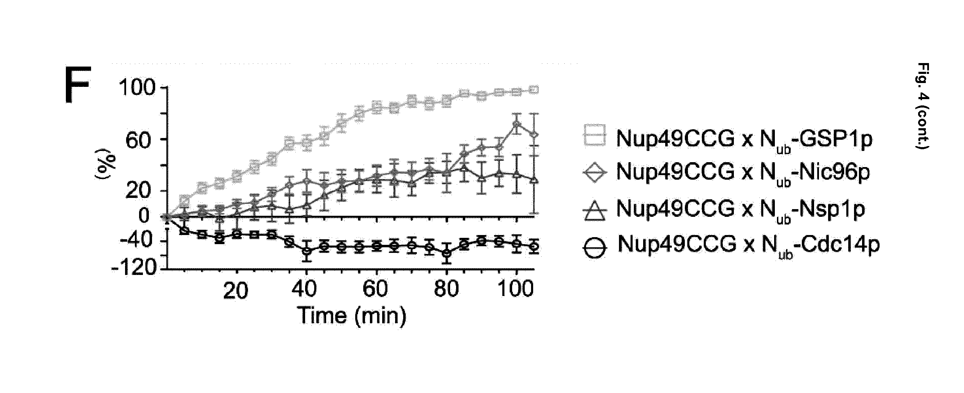

**Figure 4:** Protein interactions at the nuclear pore. (A) Overview of the structure of the nuclear pore, the Nic96p sub-complex, and the nucleo-cytoplasmic traffic in yeast. (B) Selected frames of the time-lapse analysis of a cell (white frame) expressing Nup49CCG and Nub-Gsp1 p after mating. The haploid cells are not framed. (C) Time dependent relative fluorescence intensity of Cherry (circles), GFP (squares), and the calculated fraction of converted Nup49CC (triangles) from the experiment shown in (B). Time 0 indicates the time point shortly before the fusion of the two nuclei. (D) As in (B) but showing a diploid cell expressing Nup49CCG and Nub-Cdc14p after mating. (E) Analysis as in (C) but of the experiment shown in (D). The nuclear protein NubCdc14p does not interact. (F) Time-dependent change of the fractions of converted Nup49CC through interaction with Nub-Gsp1 p (squares), Nub-Nic96p (diamonds), Nub-Nsp1 p (triangles) and Nub-Cdc14p (circles). Shown are the averages of n > 5 independent matings (error bars, s.e.). The expressions of the $N_u$b-fusions were induced by 100 μM copper. Scale bar, 5μm.

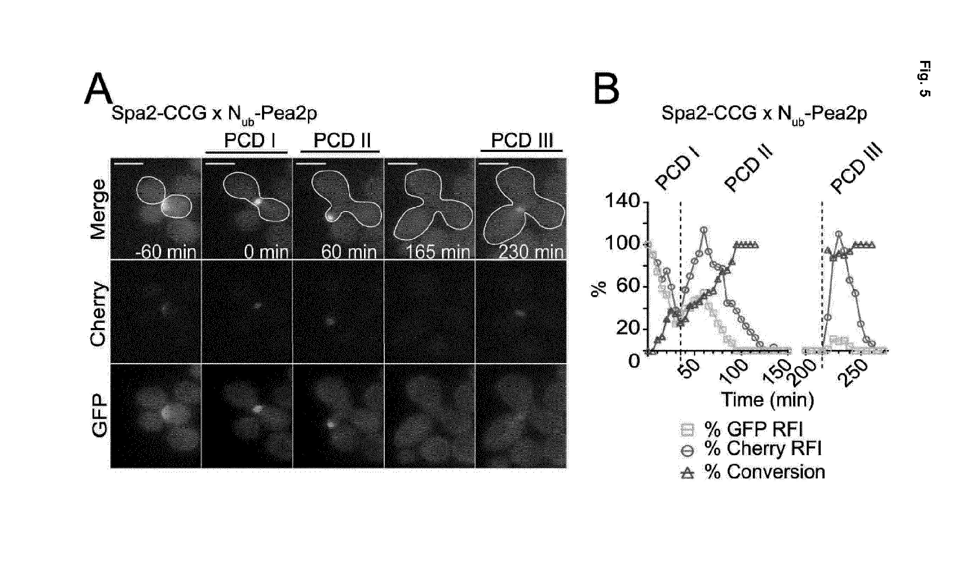

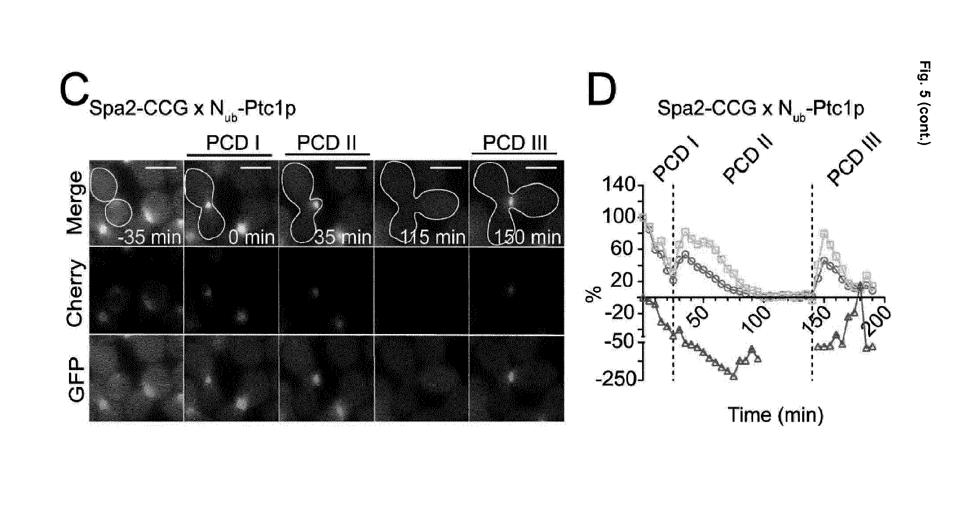

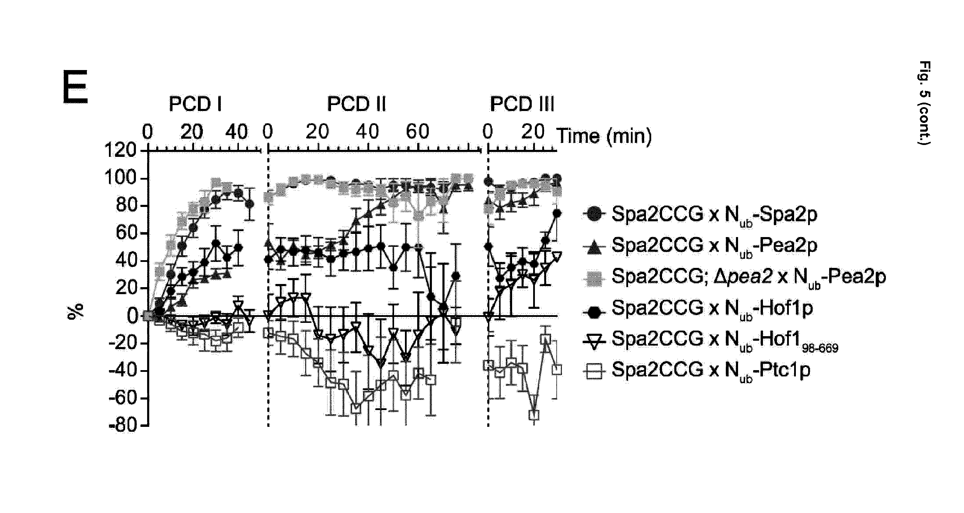

**Figure 5:** SPLIFF analysis of the interactions between components of the polar cortical domain. (A) Selected frames of the time-lapse analysis of a cell (white frame) expressing Spa2CCG and $N_u$b-Pea2p after mating. The haploid cells are not framed. PCDI indicates the stained region below the membrane of the shmoo tip, PCDII the region below the membrane of the growing bud, and PCDIII the region of cell separation. (B) Time-dependent relative fluorescence intensity (RFI) of Cherry (circles), GFP (squares), and the calculated fraction of converted Spa2CC (triangles) of the experiment shown in (A). Time 0 indicates the time point shortly before the fusion of the cells. (C), (D) as (A), (B) but with diploid cells expressing Spa2CCG and Nub-Ptc1p after mating. The cytosolic protein $N_u$b-Ptc1p does not interact with Spa2CCG. (E) Time dependent change of converted Spa2CC through interaction with $N_u$b-Spa2p(circles), Nub-Pea2p (upward triagles), $N_u$b-Pea2p in cells lacking the native Pea2p (closed squares), $N_u$b-Hof1p (hexagons), Nub-Hof198-669 (downward triangles), and Nub-Ptc1p (open squares). Dashed lines separate the analyses of the subsequent PCDs. Shown are the averages of *n* > 5 independent matings (error bars, s.e.). Scale bar, 5μm.

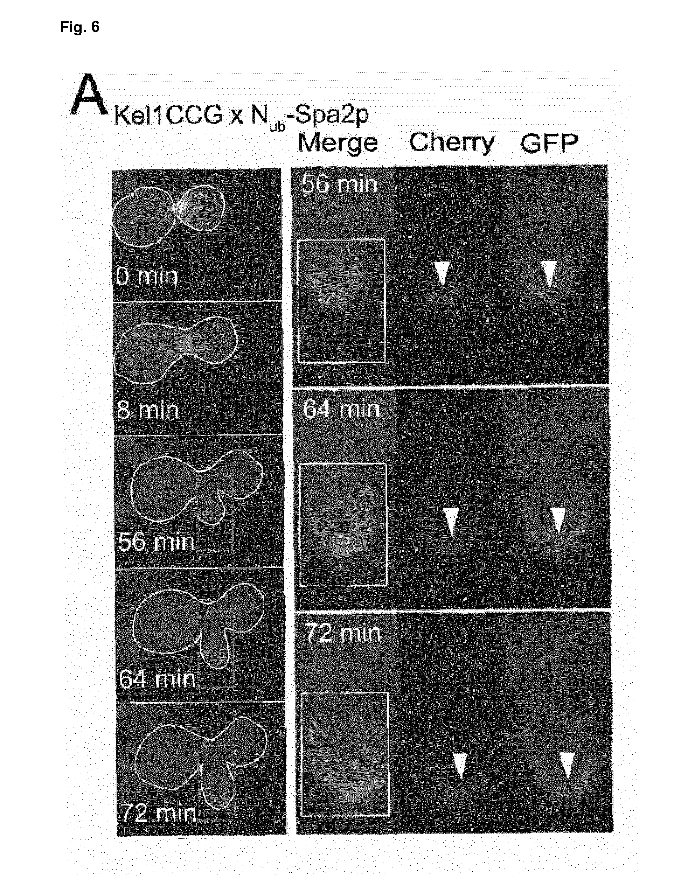

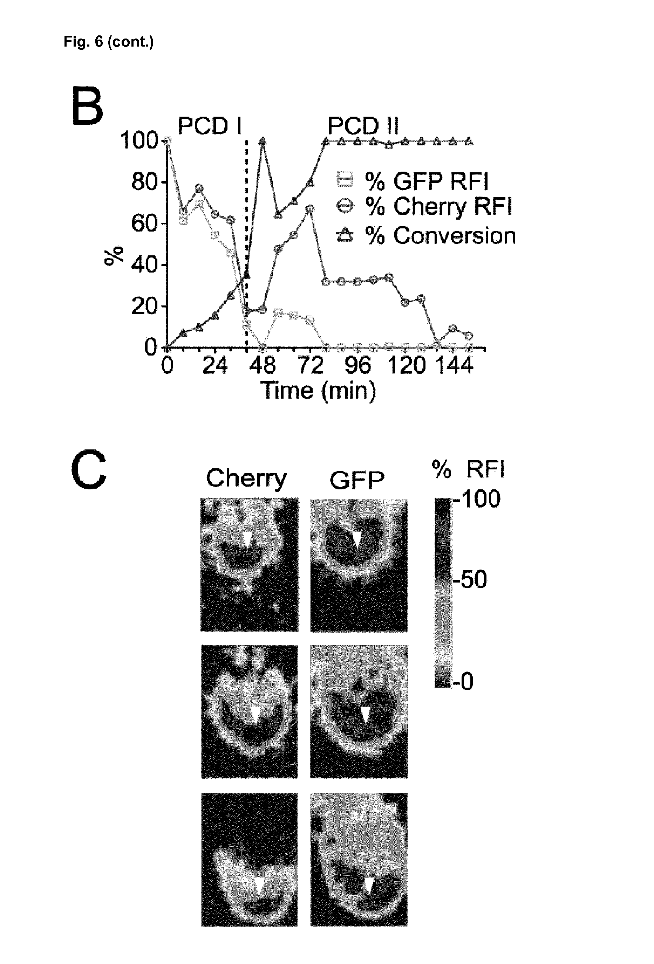

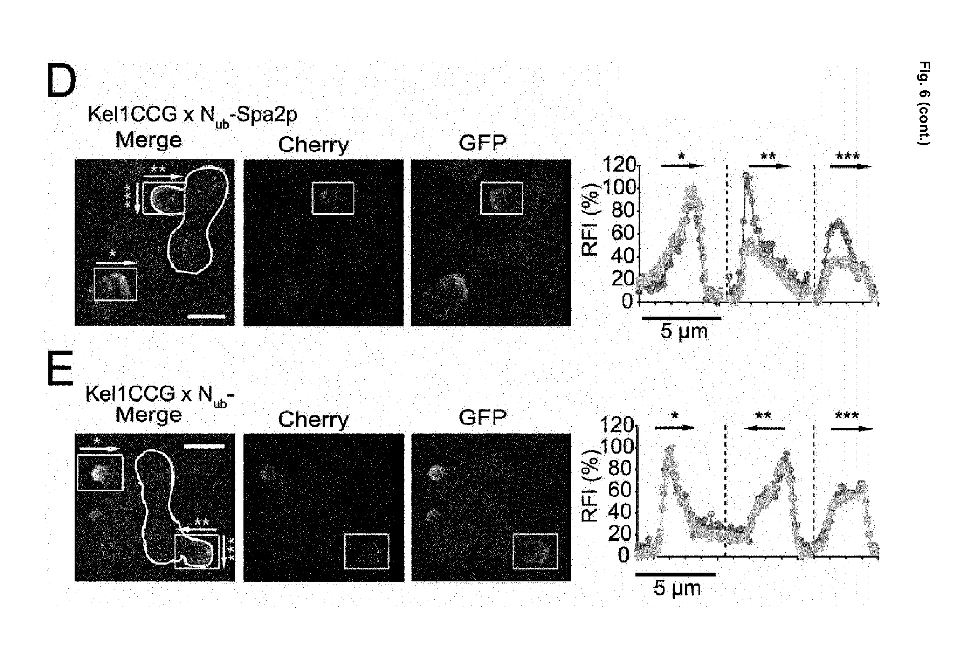

**Figure 6:** Map of the cellular location of the Spa2p/Kel1p interaction. (A) Left panel: Selected frames of the time-lapse analysis of a diploid cell (white frame) expressing Kel1 CCG and Nub-Spa2p after mating. Shown is the distribution of Kel1CCG and Kel1CC. Right panel: Blow-ups of the purple-framed buds showing the distribution of GFP (Kel1CCG) and Cherry (Kel1CCG + Kel1CC) at the indicated times. (B) Time dependent change of the relative fluorescence intensity (RFI) of Cherry (circles), GFP (squares), and the calculated fraction of converted Kel1CC (triangles) in the experiment shown in (A). Time 0 indicates the time point shortly before cell fusion. (C) Colour-coded maps of the RFIs of cherry (Kel1CCG + Kel1CC) and GFP (Kel1CCG) obtained from the area within the white squares of (A), right panel. White arrows point to identical regions of the bud in the two channels and mark regions of relative depletion of GFP (Kel1CCG). (D) Left panels: 3-D reconstructions of confocal images from GFP-, Cherry- and merged channels of yeast cells expressing Kel1CCG, or Kel1CCG and $N_u$b-Spa2p after mating (white frame). Right panels show the profiles of the relative GFP- and Cherry-intensities from the yellow- (diploid) and white-framed (haploid) buds obtained by summing over all fluorescence intensities encountered orthogonal to the direction of the respective arrows. Arrow *: across the tip of the bud of haploid cells. Arrow **: from the tip to the base of the bud of diploid cells. Arrow ***: across the tip of the bud of diploid cells. (E) Analysis as in (D) but of cells expressing Kel1CCG and a $N_u$b-fusion not binding to Kel1p. Scale bar, 5$\mu$m.

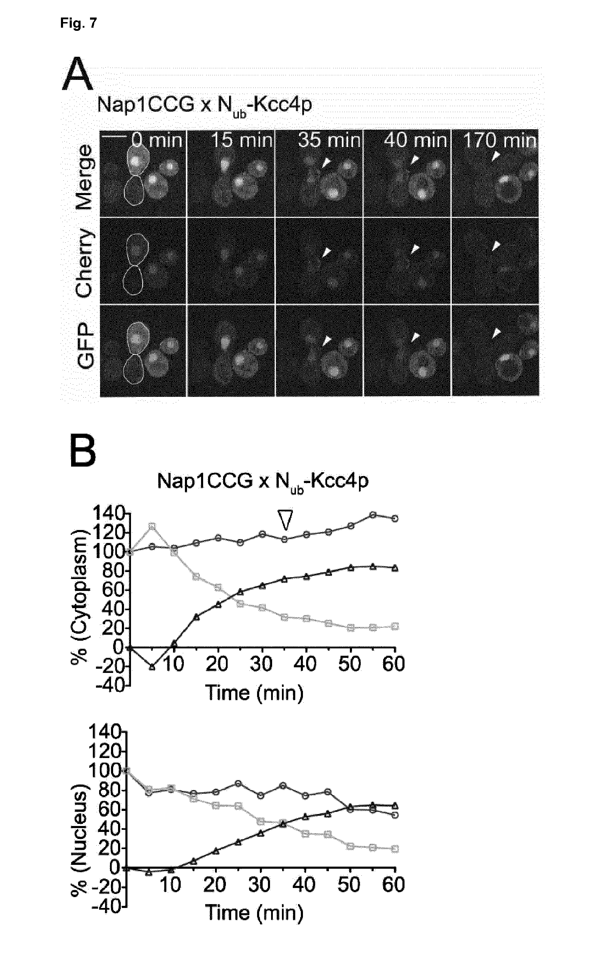

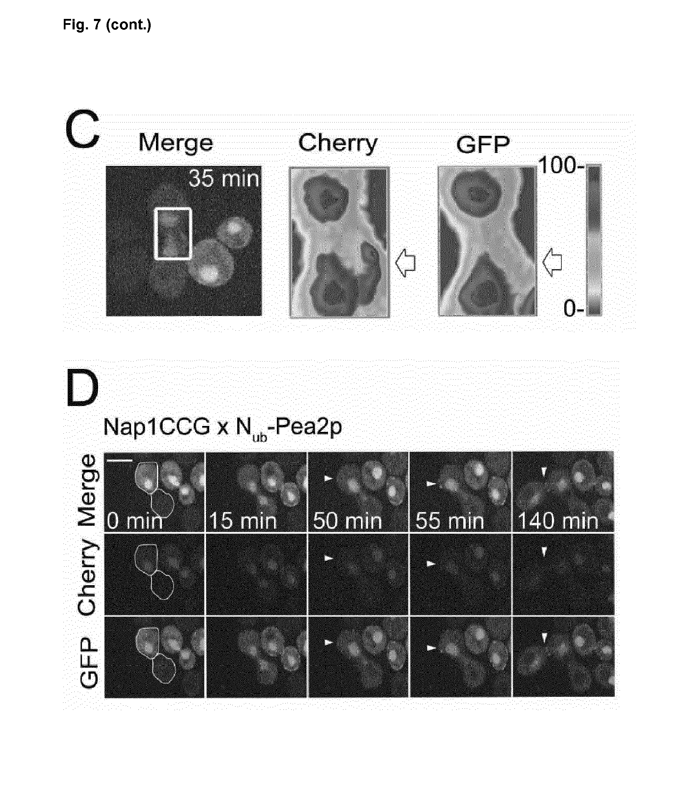

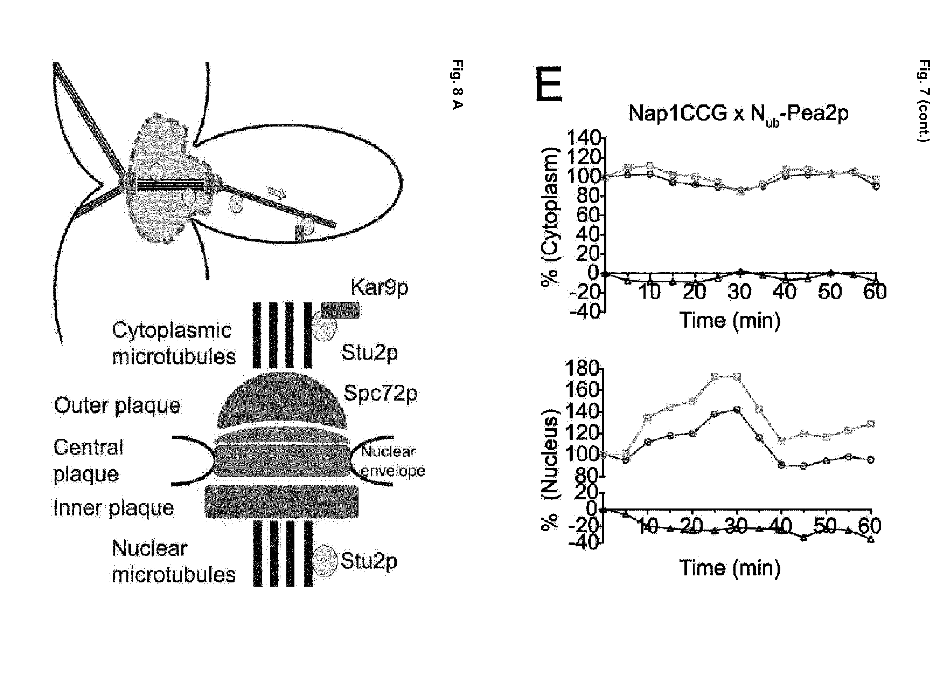

**Figure 7:** Dynamic map of the Nap1 p/Kcc4p interaction. (A) Selected frames of the time-lapse analysis of a diploid cell (white frame) expressing Nap1CCG and $N_u$b-Kcc4p after mating. White arrowheads point the region of high interaction activity at the site of a newly emerging bud. (B) Time dependent change of the relative fluorescence intensity (RFI) of Cherry (circles), and GFP (squares), as well as the calculated fraction of converted Nap1CC (triangles) in the experiment in shown (A). The analysis distinguishes between the cytoplasmic (upper panel) and the nuclear-localized Nap1CCG (lower panel). Note that the reaction occurs faster in the cytosol than in the nucleus. The white arrowhead indicates the time of the first appearance of the region of high interaction activity in the emerging bud. (C) Colour coded map of the RFI of Cherry (Nap1CCG + Nap1 CC) and GFP (Nap1 CCG) in the white-boxed cell (left panel) in the experiment shown in (A) at 35 min. White arrows point to the emerging bud. (D, E) Analysis exactly as in (A, B) but with cells co-expressing Nap1CCG and Nub-Pea2p. Nub-Pea2p does not interact with Nap1CCG. White arrowheads point to the emerging bud that is not preferentially stained by Cherry. Expressions of the $N_u$b-fusions were induced by 100 $\mu$M copper. Scale bar, 5$\mu$m.

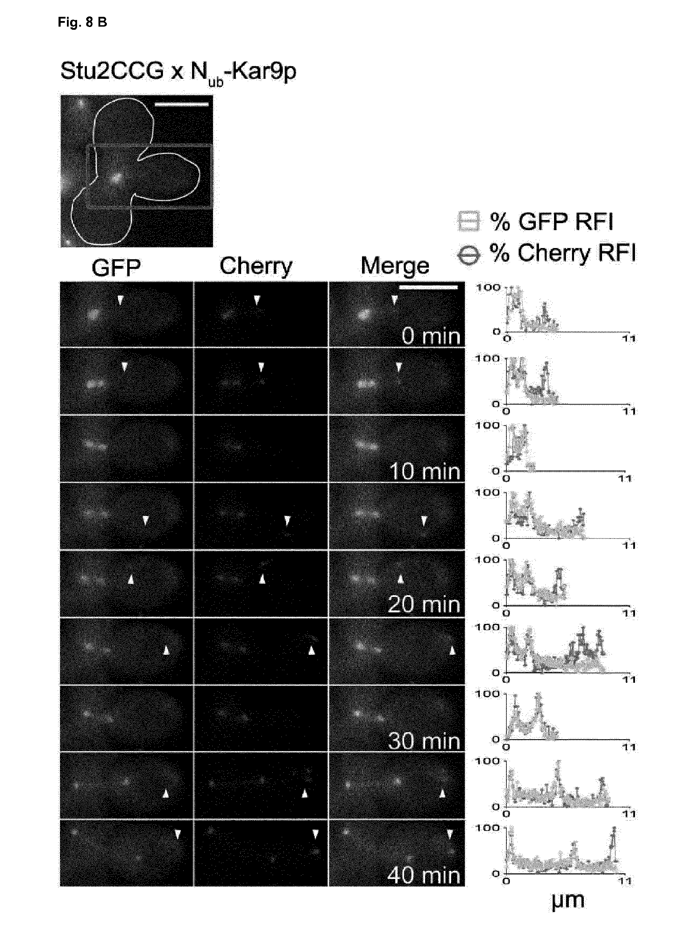

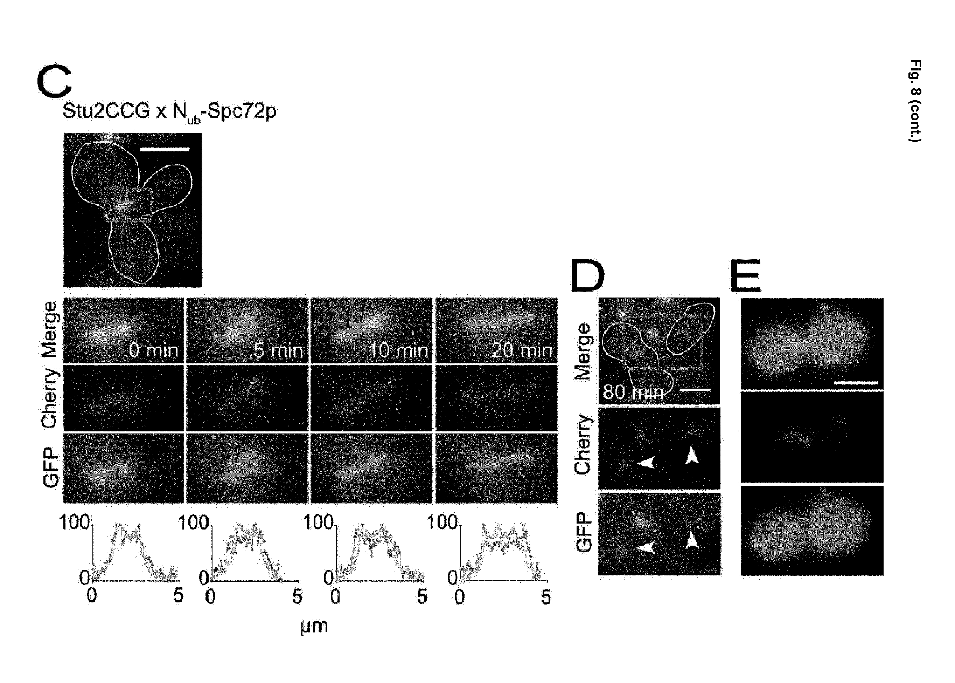

**Figure 8:** Dynamic map of the interactions of Stu2p. (A) Overview of the microtubule-based structures in yeast. (B) Upper panel: Merge of GFP and Cherry channels of a Stu2CCG-and $N_u$b-Kar9p-expressing diploid cell (white frame). Purple rectangle indicates the section shown in the frames of the time-lapse analysis of the lower left panel. Lower left panel: Selected frames of the GFP-, Cherry-, and the merged channels of the time-lapse analysis of a diploid cell expressing Stu2CCG and NubKar9p after mating. Lower right panel: Time-and position-dependent change of the RFIs of Cherry (Stu2CCG + StuCC) (circles), and GFP (Stu2CCG) (squares). Values are plotted along the distance of the microtubules connecting both SPBs, and the SPBs with the tip of the microtubules in the bud. The tip of the bud-directed microtubule is a region of preferred interaction between Stu2CCG and Nub-Kar9p. (C) Upper panel: Merge of GFP and Cherry channels of a Stu2CCG- and $N_u$b-Spc72p-expressing diploid cell (white frame) after mating. The purple rectangle indicates the section shown in the frames of the time-lapse analysis of the lower panel. Lower panel: Time- and position-dependent change of the RFIs of Cherry (circles), and GFP (squares). Values are plotted along a line connecting the two SPBs. Both ends of the structure mark sites of preferred Stu2CCG/Spc72p interaction. (D) Same cell as in (C) but after cytokinesis has already occurred. Arrowheads indicate the SPBs of mother and daughter cell (white frame). The SPB of the mother cell retains a small amount of Stu2CCG. (E) Stu2CCG- and Nub-Spc72p-expressing diploid cells after cultivation for several generations. Scale bar, 5$\mu$m.

## EXAMPLES

The examples provided herein represent practical support for particular embodiments of the invention and are not intended to limit the scope of the invention. The examples are to be considered as providing a further description of possible and potentially preferred embodiments that demonstrate the relevant technical working of one or more non-limiting embodiments.

The experiments are carried out in yeast, as the examples aim to introduce SPLIFF and to demonstrate its strength. Yeast is an ideal model system for understanding cellular behaviour by systems biology approaches and has a rich history in introducing novel technologies and concepts. All cellular components required for SPLIFF are operational in higher eukaryotic cells and thus the method should be easily adaptable in other expression systems, such as mammalian cells or cell culture.

References to green or red in the context of describing information in the figures are to be understood as GFP and Cherry, respectively.

### Rational and design of measurements

To create a robust and sensitive fluorescent reporter for protein interactions a Split-Ub module was designed, where Cub is sandwiched between, or flanked by, two spectrally different fluorescent proteins (Cherry-Cub-GFP, CCG) (Fig. 1A). Coupling CCG to the Cterminus of protein Y (Y-CCG) reveals the cellular localization of the fusion protein. Interaction of Y-CCG with a Nub-coupled interaction partner X will result in the cleavage of GFP from Y-CCG to create Y-CC. The liberated GFP is subsequently degraded. As Cherry stays attached to Y, the ratio of red to green fluorescence serves as a ratiometric reporter of protein-protein interactions and constitutes the actual readout of SPLIFF. To improve the spatial and temporal resolution of the assay, we defined the start of each reaction by fusing two yeast cells of opposite mating type, each expressing one half of the Split-Ub sensor (Fig. 1 B). The conversion of a Cherry/GFP-labelled (Y-CCG) to a Cherry-labelled fusion protein (Y-CC) is then recorded online by two-channel time-lapse fluorescence microscopy. This strategy is borrowed from chemical stopped-flow experiments where the kinetics of chemical reactions is recorded after forcing the reagents into a single chamber. In the following experiments we demonstrate the applicability of SPLIFF by investigating protein interactions that occur at different cellular locations over widely different time periods and by providing examples of successful transitions from large scale to single-cell analysis

### Interactions in the nucleus

The interaction between the nucleolar protein Net1 p and the NAD-dependent histone deacetylase Sir2p exemplifies the experimental approach (Straight et al., 1999) (Fig. 2). The CRU or CCG modules were attached in frame behind the ORF of *NET1* by homologous recombination. The Nub-module was fused 5' to the *SIR*2 ORF in $\alpha$-cells. The expression of the Nub-fusion was controlled by the *PCUP1* promoter and could be adjusted by varying the levels of copper in the medium. The growth on 5-FOA of cells co-expressing Net1CRU and Nub-Sir2p revealed the interaction in the chosen configuration of Nub- and Cub-attachment (Fig. 2B). Subsequent time-lapse microscopy of the diploid cells originating form the mating of Net1 CCG- and NubSir2p expressing $\alpha$- and $\alpha$-cell visualized the course of interaction in the nucleus from the $\alpha$-cells. The reaction was completed within 20 minutes even before nuclear fusion has occurred (Fig. 2C, D).

To test the workflow for transferring interactions revealed by large-scale interaction experiments into single cell analysis we screened Net1CRU against an array of 382 different Nub-fusions ($N_u$b-array) (Hruby et al., 2011 Among others Ubc9p, Cdc14p, and Fkh1 p were identified as further Net1p-binding partners (Table I) (Visintin et al., 1999). The interactions of Net1 p with all three Nub-fusions were subsequently analyzed by SPLIFF. The kinetic profiles of the Net1 CCG conversions were very similar to the profile induced by Nub-Sir2p. Nub-Pea2p, a Nub fusion that was not identified by the large-scale experiment, did also not interact in the SPLIFF analysis with Net1CCG. Nub-Cdc14p induced a slightly but significantly slower conversion of Net1CCG than Nub-Sir2p. To identify the rate-limiting steps of these reactions we compared the accumulation of GFP-Cdc14p in the nuclei of $\alpha$-cells with the Nub-Cdc14p- induced Net1CCG conversion (Fig. 3). In the first 10 min after mating the kinetic profiles of both reactions are similar. However, whereas the best fit of the Net1 CCG conversion is described by a sigmoid curve, the nuclear accumulation of GFP-CDC14p proceeded linear and consequently slower (Fig. 3B). We conclude that the conversion of Net1CCG to Net1CC is not limited by the association rate of the fusion proteins and the subsequent Ub-assembly and degradation of the attached GFP. The sigmoid shape indicates that the interaction between Cdc14p and Net1 p is dynamic. Nub-Cdc14p exchanges binding partners and thereby catalytically converts Net1CCG into Net1CC.

### Interactions at the nuclear pore: Slow exchange and transient interactions.

The nuclear pore complex of yeast consists of more than 30 different proteins that are organized into three different layers. Integral membrane proteins (POMs) anchor a ring of coat nucleoporins followed by adaptor nucleoporins. The adaptors position the channel nucleoporins to regulate the transfer of cargo between the nucleus and the cytoplasm (Fig. 4A) (Aitchison and Rout, 2012). Nup49p and Nic96p together with Nsp1 p and Nup57p form the Nic96p sub-complex (Fig. 4A). Nic96p is a member of the adaptor nucleoporins whereas Nup49p, Nsp1 p and Nup57p belong to the FG repeat-bearing channel nucleoporins. FG repeats interact transiently with importins and exportins that are bound to cargo and the Ran-GTPase Gsp1 p during their shuttle across the pore (Fig. 4A) (Aitchison and Rout, 2012). After mating, the nuclear membranes of the two yeast nuclei fuse and the nuclear pore complexes from both cells mix (Bucci and Wente, 1997). According to the growth rate of the coexpressing diploids Nup49CRU interacted strongly with Nub-Nup57p, -Nic96p, - Nsp1 p, and only very weakly with Nub-Gsp1 p. We measured the kinetic profiles of these interactions with Nup49p as CCG-fusion. The SPLIFF analysis of Nup49CCG visualized for the first time the interaction between a RanGTPase and a FG-repeat protein in a living cell (Figs. 4B, C, F). Nub-Gsp1 p converted Nup49CCG much faster than the Nub-fusions of Nic96p or Nsp1 p (Fig. 4F). The nuclear resident Nub-Cdc14p did not interact with Nup49CCG (Fig. 4D, E). Contrary to the transport factors, constitutive members like Nsp1p or Nic96p are known to be stably incorporated into the nuclear pore complex (Rabut et al., 2004). We therefore surmise that the slow exchange of the unlabelled proteins against the corresponding Nub-fusions of Nsp1 p or Nic96p is rate limiting for the interaction with Nup49CCG. Notably, the interaction between Nup49CCG and its Nub-labelled interaction partners started only after nuclear fusion was completed.

### Interactions between components of the polar cortical domain: Tracking interactions during the cell cycle

The proteins of the polar cortical domain (PCD) form a protein network below the plasma membrane. The composition of the PCD in yeast is not fully defined and highly dynamic (Gao et al., 2011 The PCD is located at the mating projection (PCDI), at the site of bud growth (PCDII), and at the site of cytokinesis (PCDIII). During transitions at specific points of the cell cycle, the components of the PCDs are dissolved before they are recruited to their new site (Fig. 5A). Measuring the interactions between members of the PCD thus requires tracking the CCG-fusion in their different locations during the cell cycle. Spa2p is member of the polarisome complex that organizes the actin cytoskeleton at the PCD (Sheu et al., 1998). We first identified the pool of Nub-labelled interaction partners by mating a Spa2CRU expressing strain against the Nub-array and selected a subset of those for single cell analysis (Table I). Among the found binding partners was a further member of the polarisome Nub-Pea2p (Sheu et al., 1998). Time-lapse microscopy of the mated Spa2CCG and $N_u$b-Pea2p expressing cells revealed for the first time that both proteins interact throughout the cell cycle (Fig. 5A, B, E). The specificity of the measurements was confirmed by co-expressing Spa2CCG and Nub-Ptc1p, a Nub-fusion that, according to the large-scale analysis, does not bind to Spa2CRU (Fig. 5C, D, E).

Spa2p forms homo-oligomers (Table I). Our SPLIFF analysis revealed that this reaction occurred much faster than the Spa2p-Pea2p heteromerization (Figs. 5E). Why is the Spa2p-Pea2p interaction slower? We presumed that the continuous presence of the unlabelled Pea2p in the polarisome might effectively hinder Nub-Pea2p from binding and converting Spa2CCG. Consequently, cells lacking unlabelled Pea2p should display a faster interaction. Consistent with our hypothesis we observed a 5.3 fold increase in the rate of Nub-Pea2p induced Spa2CCG conversion upon deleting the chromosomal *PEA2* in the Spa2CCG expressing a-cells (Fig. 5E). The ratio of the initial rates of conversion $RC_{iPEA2}$ to $RC_{i\Delta pea2}$ ($\Delta f = 5.3$) provides a quantitative measure. By using simplifying assumptions we calculated the fraction of bound Spa2p (Fb) by Fb = $(\Delta f1)/\Delta f$. Based on this calculation we estimate that only 19% of the Spa2CCG molecules in wild-type cells are free to react with Nub-Pea2p. The remaining 81 % (Fb) of the binding sites are occupied by endogenous Pea2p.

We identified Hof1p, a member of the cytokinesis machinery, as a new ligand of Spa2p (Table I) (Lippincott and Li, 1998; Meitinger et al., 2011). The SPLIFF analysis revealed that Hof1 p already interacts with Spa2 at the PCDI during cell fusion. Furthermore, the kinetic profile of the interaction between Spa2CCG and Nub-Hof1 p differed strikingly from the profile of the Spa2p/Pea2p interaction in tracing interactions during fusion and cytokinesis but not during bud growth (Fig. 5E). An allele of *HOF1* lacking the N-terminal FCH domain (NubHof198-669) added an interesting mechanistic detail to the understanding of this protein interaction. In contrast to the full-length protein, Nub-Hof198-669 converted Spa2CCG only during cytokinesis but not during cell fusion (Fig. 5E). The results thus allude to different modes and degrees of interaction between Hof1p and Spa2p during the three phases of polar growth.

### Mapping the cellular space of protein-protein interactions

Many proteins simultaneously occupy different locations in the cell. Each of these locations might reflect an altering set of binding partners. We examined the cellular distributions of four different protein interactions to demonstrate the spatial resolution of SPLIFF. The Nub-fusion of Kel1p was found as interaction partner of Spa2CRU (Table I). Kel1p is involved in cell fusion during mating (Philips and Herskowitz, 1998). Kel1CCG is spread across the entire bud during its growth (Fig. 6A). To specify the regions of Kel1 p-Spa2p interaction we mated Kel1CCG- with Nub-Spa2p expressing cells. Time-lapse analysis of the diploids revealed that the interaction occurs not only during cell fusion but also during bud growth (Fig. 6A, B). We analyzed the spatial distribution of the red and green fluorescence in medium-sized buds (Fig. 6C, D, E). Both intensities matched closely at the base of the bud yet segregated at its tip (Figure 6C, D, E). The reconstituted differential interaction maps describe a gradient of interactions that peak at the bud tip and trail off towards the mother cell (Fig. 6D, E).

Nap1 p is a multifunctional protein that is involved in the transport and assembly of histones and the formation of the septin ring at the bud neck of the cells (Mortensen et al., 2002; Ohkuni et al., 2003). Nap1 p is localized in the cytosol, the nucleus, and the bud neck. We recently confirmed $N_u$b-Kcc4p as binding partner of Nap1 CRU (Hruby et al., 2011). Kcc4p is a septin-localized protein kinase (Barral et al., 1999; Okuzaki and Nojima, 2001). To find out exactly when and where the interaction between Nap1 p and Kcc4p occurs we monitored the $N_u$b-Kcc4p-induced conversion of Nap1CCG in the cytosol as well as in the nucleus. The interaction started immediately after cell fusion (Figures 7A, B). The slower decrease of nuclear versus cytosolic green fluorescence indicated that Kcc4p interacts with Nap1 p primarily in the cytosol (Fig. 7B). Continuous time-lapse analysis of Nap1CCG identified at a later time point during early bud formation a red fluorescent zone devoid of green fluorescence beneath the incipient bud site (Fig. 7A, C). This region of preferred complex formation contrasted with the nucleus as well as the cytosol as two compartments of comparatively weak Nap1 p-Kcc4p interaction activity (Fig. 7C). 50 min after mating a decrease of the nuclear Cherry signal might indicate a trapping of Nap1CCG in the cytosol through the continuous co-expression of Nub-Kcc4p (Fig. 7B). Nub-Pea2p is also concentrated at the incipient bud site but does not interact with Nap1 CRU. Consequently, NubPea2p did not induce a similar dissociation of red from green fluorescence in Nap1CCG-expressing cells (Fig. 7D, E).

Stu2p is a microtubule-binding protein that is associated with the cytosolic and nuclear portion of the spindle pole body (SPB), and the nuclear, and astral microtubules (Fig. 8A) (Al-Bassam et al., 2006; Usui et al., 2003) (Kitamura et al., 2010; Winey and Bloom, 2012). The large-scale Split-Ub assay of Stu2CRU identified among others the known binding partner Kar9p, and Spc72p (Table I) (Liakopoulos et al., 2003; Miller et al., 2000; Usui et al., 2003). Mating of cells expressing Stu2CCG and the corresponding Nub-fusion proteins allowed us to dissect the microtubule structures into zones of differential Stu2p interaction activities (Fig. 8B, C). The interaction between Stu2CCG and its two $N_{ub}$-labelled binding partners started immediately upon nuclear fusion and the accompanying alignment of the two SPBs. Later during the cell cycle the unequal distribution of the red and green fluorescence across the Stu2CCG-stained microtubules clearly identified the tip of the astral microtubules as preferred site of Stu2p-Kar9p interaction (Fig. 8B). In contrast, time-lapse analysis of the cells co-expressing Stu2CCG and Nub-Spc72p revealed a preferential conversion of Stu2CCG to Stu2CC at both tips of the spindle (Fig. 8C). One hour later a small fraction of green fluorescent Stu2CCG still remained in the mother whereas the SPB of the daughter cell was stained completely red (Figure 8D). Mated cells that were cultivated for several generations displayed a complete conversion of Stu2CCG into Stu2CC (Figure 8E).

### *Discussion of the experimental results of the examples of the invention*

Due to their indifference in regard to order, location and timing, large-scale protein interaction networks are still a largely untapped resource of information that waits to be fully exploited by cell biologist (Alexander et al., 2009; Deeds et al., 2012; Kim et al., 2006; Przytycka et al., 2012; Vidal et al., 2011 Up to now dynamic and spatial features of protein interactions could only be revealed by fluorescence resonance energy transfer between two fluorescently tagged proteins or fluorescence cross correlation spectroscopy of two freely diffusing cytosolic proteins (Bacia and Schwille, 2007; Slaughter et al., 2011 However, both methods generally require careful optimizations before a positive hit from one of the high-throughput studies can be visualized as protein interaction in the cell. A transfer of large-scale interaction data into the analysis of their dynamic and spatial aspects is thus hardly possible (Lowder et al., 2011). We routinely achieved these transitions by replacing a large-scale compatible version of the Split-Ub technique by SPLIFF to subsequently probe the detected interactions with high spatial and temporal resolution in living cells.

Among the further advantages of SPLIFF is the nearly instantaneous detection of binding once the Nub- and Cub-fusion are free to interact. The Cherry signal that remains at the protein identifies the place of interaction and serves as internal reference for the decrease in GFP signal. The GFP signal reflects the fraction of uncut Cub-fusion proteins and by extrapolation the unbound fraction of the protein with regard to its Nub-labelled binding partner. As the cleavage of GFP is the temporally delayed consequence of interaction, the localization of the Cherry-Cub fusion protein can only be an approximation of where the interaction has originally occurred. To set closer limits for the preferred sites of interaction or for visualizing gradients of interactions across the cell it is thus obligatory to analyze the signals of both, the bound and unbound fraction of the Cub-fusion. A realistic interpretation of the relative green and red fluorescence intensities further necessitates a continuous on-line detection once Nub- and Cub-fusions encounter each other. We used cell fusion by mating as a controlled way of mixing the two fusion proteins. The procedure unambiguously defines the start of the reaction and allows tracking it throughout the cell cycle. This feature also enhances the reproducibility of the measurements as averaging different single cell measurements with slightly shifted cell cycle phases might obscure specific aspects of the interactions. For example, any static or unsynchronized analysis would have missed our discovery that the interaction between Hof1p and Spa2p is restricted to cell fusion and cell separation but does not occur during bud growth. Together with Sho1p, Hof1p is now the second member of the HICS-complex that is involved in cell fusion and cytokinesis (Labedzka et al., 2012). The questions whether further members of the cytokinesis network also participate in cell fusion as part of identical or different protein complexes can now be systematically investigated by SPLIFF.

The feature of simultaneously detecting bound and unbound fraction was instrumental in localizing the interaction between the microtubule-nucleator Stu2p and the component of the SPB Spc72p. The prevalence of unbound Stu2CCG at the nuclear microtubules and the high fraction of bound Stu2CC at both tips of the spindle identified the SPB as the preferred site of Stu2p-Spc72p interaction. As the converted Stu2CC dissociated from Nub-Spc72p its newly acquired localizations during later time points blurred the assignment of its interaction to a specific place in the cell. Ultimately, once conversion of Stu2CCG into Stu2CC was nearly completed the Stu2p-containing structures were uniformly stained by Cherry and the localization of interaction not longer feasible.

The moderate affinity between Nub and Cub might stabilize transient or weak protein complexes by reducing the rate of their dissociation (Müller and Johnsson, 2008). Applying the rate of CCG to CG conversion and not the CCG/CG ratio as the preferred measure to specify the cellular place and duration of a certain interaction should avoid that this temporal trapping might interfere with the spatial and temporal resolution of SPLIFF.

The invention shows that the rapid and continuous detection of interaction, the detection of the unbound fraction, and the ability to control the start of the reaction as three requirements for an optimally accurate SPLIFF-analysis of protein interactions. Split-protein sensors lacking any of these features call for an even more deliberate interpretation with regard to localizing and timing a certain protein interaction. For example, the fluorescence signal generated upon interaction in Split-GFP (BiFC) might simply reflect the localization of the binding partner with the strongest localization signal and not necessarily the cellular site of their interaction (Kerppola, 2006; Stynen et al., 2012).

Despite the limitations of SPLIFF in exploring quantitative aspects of protein interactions we propose that the calculated occupancy (Fb) of a protein is a useful quantitative reference for classifying protein complexes. For example, the Fb of 81 % indicates that the Spa2p/Pea2p complex is much more stable than the Net1 p/Fkh1 p complex as the absence of unlabelled Fkh1p revealed no significant increase in the respective RCi of Net1 CCG (Ghaemmaghami et al., 2003). The application of SPLIFF in higher eukaryotes is also possible, as all essential components of this technique are also functional in these cells (Pratt et al., 2007).

*Materials and methods*

**Time-lapse microscopy**. Time-lapse experiments were carried out using a DeltaVision fluorescence microscope (Applied Precision) provided with a steadystate heating chamber and equipped with a mercury arc lamp and a camera CoolSNAP HQ2-ICX285 (Photometrics). In all cases a 100x NA 1.4 UPlanSApo oil immersion objective (Olympus) was used. Unless otherwise stated, images at 0.8-$\mu$m intervals for a 4-section z stack were collected in 5 minutes intervals on three channels: Brightfield, GFP (excitation 470/40nm and emission 525/50) and Cherry (excitation 572/35nm and emission 632/60). The CCD capture time was adapted to the intensity of GFP and Cherry signal in every construct to reduce bleaching and photo toxicity.

**Confocal microscopy.** Confocal microscopy was conducted using An Axio Observer Z.1 Spinning Disc microscope (Zeiss). Images were acquired under the control of the AxioVision 4.8.2 software (Zeiss) as Z-series with fifteen to twenty slices. The distance between two Z-slices was set to 260 nm. For image acquisition, a PlanAPOCHROMAT 63X/1.4 Oil DIC ∞/0.17 objective and 1x1 binning was used. The camera gain was set to 1. For excitation of GFP or Cherry a 488 nm diode laser at 80% laser power or a 561 nm diode laser at 100% laser power was used, respectively.

**Fluorescence quantification and analysis.** All files generated by the microscope software (Resolve3D soft-WoRx-Acquire Version: 4.0.0 Release 16) were processed and analyzed with ImageJ 1.45s (US National Institutes of Health, http://imagej.nih.gov/ij/download.html). Z-stacks were projected onto two-dimensional images. In order to quantify the fluorescence over time we selected regions of interest (ROI) and a region within the cell that could serve as background for the quantification. In experiments where the CCG-fusion was distributed throughout the cell a section from a haploid Nub-fusion-expressing cell occupying the same field was used as reference. To quantify localization-dependent protein interactions we analyzed the spatial fluorescence profiles of the structures of interest. Z-stacks were projected to two-dimensional images. The intensity variations of the GFP and Cherry Channels were followed on a chosen path or across a certain area of the cell using either ImageJ Plot Profile tool or Interactive 3D surface plot tool respectively.

To obtain the Relative Fluorescence Intensities (RFI) of each channel, we first calculated the fluorescence intensity FI by:

$$FI = Area(loc) \times MeanGreyValue(loc) - Area(loc) \times MeanGreyValue(back)$$

to then calculate the relative fluorescence intensities RFI by:

$$RFI(x) = \frac{FI(x) \times 100}{FI(start)}$$

(*loc*) specifies the signal of the ROI areas and *(back)* the signal derived from the background areas. In time-dependent interaction experiments (*x*) represents time. *FI(start)* represents the fluorescence intensities shortly before the fusion of the cells. The RFIs of both channels were then used to calculate the extent of the interaction (FD) as measured by conversion of the CCG-fusion to the CC-Fusion by:

$$F_D(x) = 100 \times \frac{RFI_{RED} - RFI_{GREEN}}{RFI_{RED}}$$

In localization-dependent interactions (*x*) represents distance along the chosen path. In static localization-dependent interactions *FI(start)* represents the maximal value of the analogous structure in a haploid CCG-expressing strain, which resides in the same field as the investigated diploid. In the time- and localization-dependent interactions of Stu2CCG *FI(start)* represents the maximal intensities of both channels for each time frame shown.

**Image preparation for publication.** Images were prepared for publication with Acrobat Illustrator (Adobe) using linear contrast and intensity adjustments. When needed, Delta Vision fluorescence microscope images were deconvoluted using the software Resolve3D (softWoRx-Acquire Version: 4.0.0 Release 16). 3D reconstructions were obtained from Z stacks acquired by confocal microscopy using imaged 3D projection tools.

**Interaction analysis by cell growth.** Large scale Split-Ub assays were performed as described (Dünkler et al., 2012; Hruby et al., 2011). Measuring interactions between individual Nub- and Cub-fusion proteins by spotting yeast cells expressing both fusions onto 5-FOA and SD Ura- containing media was essentially as described (Eckert and Johnsson,2003).

**Single cell interaction analysis.** The CCG- and Nub-fusion-expressing a- and acells were separately incubated in SD-medium at 30°C overnight. If not indicated otherwise cells were diluted into fresh medium without copper and grown to an OD600 of 0.6 - 1. After mixing equal amounts of the a- and a-cells (between 0.5 and 0.75 ml each), the culture was immediately spun down, and resuspended in 50 μL of SD medium. 3 μL of this suspension were immobilized by fixing a cover slide with parafilm strips on a custom-designed glass slide containing solid, non-fluorescent agar-SD without copper if not indicated otherwise. The slide was immediately incubated at 30°C under the microscope. Pictures were taken after 45 to 75 min of incubation when the first zygote formations became visible. **Construction of Nub- and Cub-fusion genes and other molecular manipulations.** *Nub*-and *Cub*-fusion genes were constructed as described (Dünkler et al., 2012; Hruby et al., 2011). Gene deletions were performed by PCR-based methods as described (Janke et al., 2004).

The switch from the CRU to the CCG module was achieved by cloning the same PRC product used for the construction of the respective CRU fusion in front the CCG-cassettes on a pRS306 or pRS304 vector. Linearization of the obtained plasmids with restriction enzymes cutting only once in the amplified sequences of the ORFs of the respective genes and homologous recombination after transformations of the yeast were as described (Dünkler et al., 2012). Successful recombination was verified by colony-PCR.

**Growth conditions, yeast strains, and genetic methods.** Culture media and yeast genetic methods were performed following standard protocols (Dünkler et al., 2012). Media for the Split-Ub interaction assay contained 1 mg/ml 5-fluoroorotic acid (5- FOA). Yeast strains JD47, JD53, and JD51 are as described (Dünkler et al., 2012).

**Calculating the initial rates of conversion.** By plotting the relative decrease in GFP fluorescence FD we calculated by linear regression the initial rates of conversion (RCi) for the first 15 min of the reaction.

**Calculating the ligand-bound fraction Fb.** We assumed that the conversion of YCCG fusion into the Y-CC fusion after mating with the Nub-X expressing strain is described by a bimolecular reaction where the rate of conversion is given by

$$\frac{dC_{Y\text{-}CCG}}{dt} = k \times C_{Y\text{-}CCGfree}(t) \times C_{Nub\text{-}Xfree}(t)$$

Y-C$_{CCG\text{-}free}$ is the concentration of unbound Y-CCG-fusion and C$_{Nub\text{-}Xfree}$ the concentration of unbound Nub-X. k is the rate constant of the reaction and a complex composite of a mix of different reactions including among others the rate of $_{Nub}$ and Cub reassembly, the cleavage rate by the ubiquitin specific proteases, the diffusion constants.

We assume that shortly after cell fusion k and C$_{Nub\text{-}Xfree}$ are very similar if not identical in the two diploids that were derived by mating of the Nub-X-expressing strain with a Y-CCG-fusion-expressing strain where the gene X was either deleted (Δx) or not (*X*). It then follows that the quotient of the initial rates:

$$\frac{dC_{Y\text{-}CCG}/dt(\Delta x)}{dC_{Y\text{-}CCG}/dt(X)} = f_\Delta = \frac{C_{Y\text{-}CCG\,free\,(\Delta X)}(t_0)}{C_{Y\text{-}CCG\,free\,(X)}(t_0)} = \frac{C_T}{(C_T - C_B)}$$

CT is the sum of the concentrations of free and bound Y-CCG-fusion whereas C$_B$ is the concentration of Y-CCG-fusion bound to X.

$$\frac{C_B}{C_T} = F_b = \frac{(f_\Delta - 1)}{f_\Delta}$$

$dC_{Y\text{-}CCG}/dt(\Delta X)$ and $dC_{Y\text{-}CCG}/dt(X)$, the RC is of both reactions were derived by calculating a linear regression of the first 15 min after the reaction has started. The averages from at least four different independent measurements were taken. An analysis of the two reactions was only performed when the curves of the kinetic profiles were significantly different.

**Statistical evaluation and curve fitting of the experimental data.** Curve fitting was performed in R version 2.15.1 (http://www.R-project.org) by minimizing the least squares deviation of a set of regression functions to the data. The best fitting function was determined by the Akaike information criterion. For comparison of two groups the sum of the residual sum of squares of the individual regressions is then compared to that of the regression for the combined data using the F-test (Motulsky and Ransnas, 1987).

**Table 1:** Interaction partners of Net1p, Spa2p and Stu2p identified by large-scale Split-Ub interaction screens.

| $C_{ub}$-fusions | $N_{ub}$-fusions |
|---|---|
| Net1p | Scc2p, Smt3p, Swi6p, Irr1p, Net1p, Fkh1p, Cdc14p, Fkh2p, Ubc9p |
| Spa2p | Kel1p, Hof1p, YmR124wp, Spa2p, Bud14p, Sec4p, Pea2p |
| Stu2p | Spc24p, Bik1p, Spc72p, Bim1p, Kar3p, Dad1p, Dad3p, Stu2p, Clb4p, Kip2p, Kip3p, Kar9p |

Additionally, further experimentation shows that the preferred embodiments of the invention provide surprising and unexpected effects, thereby solving the problem of the invention in a non-obvious fashion.

**References**

Aitchison, J. D., Rout, M. P. (2012). The yeast nuclear pore complex and transport through it. Genetics 190: 855-883.

Al-Bassam, J., van Breugel, M., Harrison, S. C., Hyman, A. (2006). Stu2p binds tubulin and undergoes an open-to-closed conformational change. J Cell Biol 172: 1009-1022. Alexander, R. P., Kim, P. M., Emonet, T., Gerstein, M. B. (2009). Understanding modularity in molecular networks requires dynamics. Sci Signal 2: pe44.

Bacia, K., Schwille, P. (2007). Practical guidelines for dual-color fluorescence cross-correlation spectroscopy. Nat Protoc 2: 2842-2856.

Barral, Y., Parra, M., Bidlingmaier, S., Snyder, M. (1999). Nim1-related kinases coordinate cell cycle progression with the organization of the peripheral cytoskeleton in yeast. Genes Dev 13: 176-187.

Breitkreutz, A., Choi, H., Sharom, J. R., Boucher, L., Neduva, V., Larsen, B., Lin, Z. Y., Breitkreutz, B. J., Stark, C., Liu, G., et al. (2010). A global protein kinase and phosphatase interaction network in yeast. Science 328: 1043-1046.

Bucci, M., Wente, S. R. (1997). In vivo dynamics of nuclear pore complexes in yeast. J Cell Biol 136: 1185-1199.

Deeds, E. J., Krivine, J., Feret, J., Danos, V., Fontana, W. (2012). Combinatorial complexity and compositional drift in protein interaction networks. PLoS One 7: e32032.

Dünkler, A., Muller, J., Johnsson, N. (2012). Detecting protein-protein interactions with the split-ubiquitin sensor. Methods Mol Biol 786: 115-130.

Eckert, J. H., Johnsson, N. (2003). Pex10p links the ubiquitin conjugating enzyme Pex4p to the protein import machinery of the peroxisome. J Cell Sci 116: 3623-3634.

Gao, J. T., Guimera, R., Li, H., Pinto, I. M., Sales-Pardo, M., Wai, S. C., Rubinstein, B., Li, R. (2011). Modular coherence of protein dynamics in yeast cell polarity system. Proc Natl Acad Sci U S A 108: 7647-7652.

Gavin, A. C., Bosche, M., Krause, R., Grandi, P., Marzioch, M., Bauer, A., Schultz, J., Rick, J. M., Michon, A. M., Cruciat, C. M., et al. (2002). Functional organization of the yeast proteome by systematic analysis of protein complexes. Nature 415: 141-147. Ghaemmaghami, S., Huh, W. K., Bower, K., Howson, R. W., Belle, A., Dephoure, N., O'Shea, E. K., Weissman, J. S. (2003). Global analysis of protein expression in yeast. Nature 425: 737-741.

Hruby, A., Zapatka, M., Heucke, S., Rieger, L., Wu, Y., Nussbaumer, U., Timmermann, S., Dunkler, A., Johnsson, N. (2011). A constraint network of interactions: protein-protein interaction analysis of the yeast type II phosphatase Ptc1p and its adaptor protein Nbp2p. J Cell Sci 124: 35-46.

Janke, C., Magiera, M.M., Rathfelder, N., Taxis, C., Reber, S., Maekawa, H., Moreno-Borchart, A., Doenges, G., Schwob, E., Schiebel, E., Knop, M. (2004) A versatile toolbox for PCR-based tagging of yeast genes: new fluorescent proteins, more markers and promoter substitution cassettes. Yeast, 21: 947-962.

Johnsson, N., Varshavsky, A. (1994). Split ubiquitin as a sensor of protein interactions in vivo. Proc Natl Acad Sci U S A 91: 10340-10344.

Kerppola, T. K. (2006). Design and implementation of bimolecular fluorescence complementation (BiFC) assays for the visualization of protein interactions in living cells. Nat Protoc 1: 1278-1286.

Kim, P. M., Lu, L. J., Xia, Y., Gerstein, M. B. (2006). Relating three-dimensional structures to protein networks provides evolutionary insights. Science 314: 1938- 1941.

Kitamura, E., Tanaka, K., Komoto, S., Kitamura, Y., Antony, C., Tanaka, T. U. (2010). Kinetochores generate microtubules with distal plus ends: their roles and limited lifetime in mitosis. Dev Cell 18: 248-259.

Krogan, N. J., Cagney, G., Yu, H., Zhong, G., Guo, X., Ignatchenko, A., Li, J., Pu, S., Datta, N., Tikuisis, A. P., et al. (2006). Global landscape of protein complexes in the yeast Saccharomyces cerevisiae. Nature 440: 637-643.

Labedzka, K., Tian, C., Nussbaumer, U., Timmermann, S., Walther, P., Müller, J., Johnsson, N. (2012). Sho1p connects the plasma membrane with proteins of the cytokinesis network via multiple isomeric interaction states. J Cell Sci. 25: 4103-4113 Liakopoulos, D., Kusch, J., Grava, S., Vogel, J., Barral, Y. (2003). Asymmetric loading of Kar9 onto spindle poles and microtubules ensures proper spindle alignment. Cell 112: 561-574.

Laser, H. et al., PNAS, Proc Natl Acad Sci U S A. 2000 Dec 5;97(25):13732-7

Lippincott, J., Li, R. (1998). Dual function of Cyk2, a cdc15/PSTPIP family protein, in regulating actomyosin ring dynamics and septin distribution. J Cell Biol 143: 1947- 1960. Lowder, M. A., Appelbaum, J. S., Hobert, E. M., Schepartz, A. (2011). Visualizing protein partnerships in living cells and organisms. Curr Opin Chem Biol 15, 781-788. Meitinger, F., Boehm, M. E., Hofmann, A., Hub, B., Zentgraf, H., Lehmann, W. D., Pereira, G. (2011). Phosphorylation-dependent regulation of the F-BAR protein Hof1 during cytokinesis. Genes Dev 25: 875-888.

Miller, J. P., Lo, R. S., Ben-Hur, A., Desmarais, C., Stagljar, I., Noble, W. S., Fields, S. (2005). Large-scale identification of yeast integral membrane protein interactions. Proc Natl Acad Sci U S A 102: 12123-12128.

Miller, R. K., Cheng, S. C., Rose, M. D. (2000). Bim1 p/Yeb1 p mediates the Kar9p-dependent cortical attachment of cytoplasmic microtubules. Mol Biol Cell 11: 2949- 2959. Mortensen, E. M., McDonald, H., Yates, J., 3rd, Kellogg, D. R. (2002). Cell cycledependent assembly of a Gin4-septin complex. Mol Biol Cell 13, 2091-2105. Motulsky, H. J., Ransnas, L. A. (1987). Fitting curves to data using nonlinear regression: a practical and nonmathematical review. Faseb J 1: 365-374.

Müller, J., Johnsson, N. (2008). Split-ubiquitin and the split-protein sensors: chessman for the endgame. Chembiochem 9: 2029-2038.

Ohkuni, K., Okuda, A., Kikuchi, A. (2003). Yeast Nap1-binding protein Nbp2p is required for mitotic growth at high temperatures and for cell wall integrity. Genetics 165: 517-529. Okuzaki, D., Nojima, H. (2001). Kcc4 associates with septin proteins of Saccharomyces cerevisiae. FEBS Lett 489: 197-201.

Philips, J., Herskowitz, I. (1998). Identification of Kel1p, a kelch domain-containing protein involved in cell fusion and morphology in Saccharomyces cerevisiae. J Cell Biol 143: 375-389.

Pratt, M. R., Schwartz, E. C., Muir, T. W. (2007). Small-molecule-mediated rescue of protein function by an inducible proteolytic shunt. Proc Natl Acad Sci U S A 104: 11209-11214.

Przytycka, T. M., Singh, M., Slonim, D. K. (2010). Toward the dynamic interactome: it's about time. Brief Bioinform 11: 15-29.

Rabut, G., Doye, V., Ellenberg, J. (2004). Mapping the dynamic organization of the nuclear pore complex inside single living cells. Nat Cell Biol 6: 1114-1121.

Sheu, Y. J., Santos, B., Fortin, N., Costigan, C., Snyder, M. (1998). Spa2p interacts with cell polarity proteins and signaling components involved in yeast cell morphogenesis. Mol Cell Biol 18: 4053-4069.

Slaughter, B. D., Unruh, J. R., Li, R. (2011). Fluorescence fluctuation spectroscopy and imaging methods for examination of dynamic protein interactions in yeast. Methods Mol Biol 759: 283-306.

Straight, A. F., Shou, W., Dowd, G. J., Turck, C. W., Deshaies, R. J., Johnson, A. D., Moazed, D. (1999). Net1, a Sir2-associated nucleolar protein required for rDNA silencing and nucleolar integrity. Cell 97: 245-256.

Stynen, B., Tournu, H., Tavernier, J., Van Dijck, P. (2012). Diversity in genetic in vivo methods for protein-protein interaction studies: from the yeast two-hybrid system to the mammalian split-luciferase system. Microbiol Mol Biol Rev76: 331-82.

Tarassov, K., Messier, V., Landry, C. R., Radinovic, S., Serna Molina, M. M., Shames, I., Malitskaya, Y., Vogel, J., Bussey, H., Michnick, S. W. (2008). An in vivo map of the yeast protein interactome. Science 320: 1465-1470.

Uetz, P., Giot, L., Cagney, G., Mansfield, T. A., Judson, R. S., Knight, J. R., Lockshon, D., Narayan, V., Srinivasan, M., Pochart, P., et al. (2000). A comprehensive analysis of protein-protein interactions in Saccharomyces cerevisiae. Nature 403: 623-627.

Usui, T., Maekawa, H., Pereira, G., Schiebel, E. (2003). The XMAP215 homologue Stu2 at yeast spindle pole bodies regulates microtubule dynamics and anchorage. Embo J 22: 4779-4793.

Vidal, M., Cusick, M. E., Barabasi, A. L. (2011). Interactome networks and human disease. Cell 144: 986-998.

Visintin, R., Hwang, E. S., Amon, A. (1999). Cfi1 prevents premature exit from mitosis by anchoring Cdc14 phosphatase in the nucleolus. Nature 398: 818-823. Winey, M., Bloom, K. (2012). Mitotic spindle form and function. Genetics 190: 1197-1224.

Wittke, S., Lewke, N., Muller, S., Johnsson, N. (1999). Probing the molecular environment of membrane proteins in vivo. Mol Biol Cell 10: 2519-2530.

Yu, H., Braun, P., Yildirim, M. A., Lemmens, I., Venkatesan, K., Sahalie, J., Hirozane-Kishikawa, T., Gebreab, F.,

Li, N., Simonis, N., et al. (2008). High-quality binary protein interaction map of the yeast interactome network. Science 322: 104-110.

**Claims**

1. Fluorescent reporter for determining molecular interactions, comprising a ubiquitin sub-domain attached to two fluorescent marker molecules, preferably flanking the ubiquitin sub-domain, whereby

   - the fluorescent marker molecules exhibit distinct fluorescent properties from one another, such as distinct absorption and/or emission spectra, and
   - one of said fluorescent marker molecules is capable of cleavage from the fluorescent reporter via ubiquitin-specific protease activity upon physical interaction between a) said ubiquitin sub-domain of the fluorescent reporter and b) a complementary ubiquitin sub-domain present attached to a separate molecule.

2. Fluorescent reporter according to the preceding claim, **characterised in that** the ubiquitin sub-domain of the fluorescent reporter and the complementary ubiquitin sub-domain are capable of physically interacting with each other to form a quasi-native ubiquitin moiety, recognisable by an ubiquitin-specific protease.

3. Fluorescent reporter according to any one of the preceding claims, **characterised in that** the ubiquitin sub-domain of the fluorescent reporter is a C-terminal sub-domain (Cub) and the complementary ubiquitin sub-domain is an N-terminal sub-domain (Nub).

4. Fluorescent reporter according to any one of the preceding claims, **characterised in that** the fluorescent marker molecule capable of cleavage from the fluorescent reporter is

   - degradable after cleavage, leading to a loss of fluorescence of said marker molecule, or
   - non-degradable after cleavage, enabling spatial separation of the two fluorescent marker molecules.

5. Fluorescent reporter according to any one of the preceding claims, **characterised in that** a protein to be investigated for molecular interaction with another molecule, said other molecule being preferably a protein, nucleic acid, small molecule, antibody, lipid or other chemically or biologically relevant molecule, for example said protein is to be investigated for one or more protein-protein interactions, is covalently attached to the fluorescent reporter, preferably as a fusion protein.

6. Fluorescent reporter according to any one of the preceding claims, **characterised in that** the fluorescent marker molecules are fluorescent proteins, such as Cherry or GFP, or fluorophores, whereby said fluorophores are preferably attached to proteins or protein domains that are fused either N- and/or C-terminally to the ubiquitin sub-domain of the reporter, for example a SNAP-Tag, CLIP-Tag and/or HALO-Tag.

7. Fluorescent reporter according to any one of the preceding claims, **characterised in that** the ubiquitin sub-domain and flanking fluorescent proteins are covalently attached as a fusion protein.

8. Fluorescent reporter according to any one of the preceding claims, **characterised in that** said fluorescent reporter is covalently attached to the N- and/or C-terminus of a protein to be investigated for physical interaction, preferably as a fusion protein.

9. Fluorescent reporter according to any one of the preceding claims, **characterised in that**

   - said fluorescent reporter comprises of Cherry fluorescent protein, Cub and green fluorescent protein (GFP), in the provided order, whereby a protein to be investigated for physical interaction is covalently attached to Cherry fluorescent protein, preferably as a fusion protein, and GFP is capable of cleavage from the fluorescent reporter via ubiquitin-specific protease activity, or
   - said fluorescent reporter comprises of Green fluorescent protein, Cub and of Cherry fluorescent protein, in the provided order, whereby a protein to be investigated for physical interaction is covalently attached C-terminally to Cherry fluorescent protein, preferably as a fusion protein, and GFP-Cub is capable of cleavage from the protein to be investigated for physical interaction via ubiquitin-specific protease activity.

10. Nucleic acid molecule encoding a fluorescent reporter or fusion protein according to any one of the preceding claims.

11. Cell comprising a fluorescent reporter or fusion protein according to any one of the preceding claims or a nucleic acid molecule encoding said reporter or fusion protein according to any one of the preceding claims.

12. System for determining physical interaction between a protein and another molecule, said other molecule being preferably a protein, nucleic acid, small molecule, antibody, lipid or other chemically or biologically relevant molecule, comprising

- a fluorescent reporter according to any one of claims 1 to 9 attached, preferably as a fusion protein, to said first protein to be investigated for physical interaction with another molecule, and
- another molecule to be investigated for physical interaction with said first protein, attached, preferably as a fusion protein, to a complementary ubiquitin sub-domain, whereby
- physical interaction between said first protein and said other molecule enables physical interaction between the ubiquitin sub-domain of the fluorescent reporter and the complementary ubiquitin sub-domain of said other molecule to form a quasi-native ubiquitin moiety, recognisable by an ubiquitin-specific protease.

13. Method for determining a physical interaction between a protein and another molecule, said other molecule being preferably a protein, nucleic acid, small molecule, antibody, lipid or other chemically or biologically relevant molecule, comprising

- providing a fluorescent reporter according to any one of claims 1 to 9 attached, preferably as a fusion protein, to said first protein to be investigated for physical interaction with another molecule, and
- providing another molecule to be investigated for physical interaction with said first protein, attached, preferably as a fusion protein, to a complementary ubiquitin sub-domain, whereby
- when physical interaction between said first protein and said other molecule occurs, preferably in a cell, physical interaction between the ubiquitin sub-domain of the fluorescent reporter and the complementary ubiquitin sub-domain of said other molecule is enabled to form a quasi-native ubiquitin moiety, recognisable by an ubiquitin-specific protease, and
- ubiquitin-specific protease activity leads to cleavage and subsequent degradation of the cleavable fluorescent marker molecule leading to a loss of fluorescence of said marker, or ubiquitin-specific protease activity leads to cleavage and subsequent spatial separation of the two fluorescent markers.

14. Method for determining the temporal and/or spatial distribution of a physical interaction between a protein and another molecule, said other molecule being preferably a protein, nucleic acid, small molecule, antibody, lipid or other chemically or biologically relevant molecule, using a method according to the preceding claim, comprising

- the use of fluorescent imaging and/or detection means, preferably two-channel time lapse fluorescent microscopy, fluorescence energy resonance transfer (FRET) or fluorescence cross correlation spectroscopy, to determine changes in fluorescence of the cleavable and/or non-cleavable fluorescent marker molecules over time and/or with regard to the spatial distribution of fluorescent signals, whereby
- a change in the ratio of fluorescence intensities of the cleavable and non-cleavable fluorescent marker molecules, preferably a reduction in fluorescence intensity of the cleavable fluorescent marker molecule in relation to the non-cleavable fluorescent marker molecule, indicates physical interaction between a first protein and another molecule, and/or
- a change in relative distance between the cleavable and non-cleavable fluorescent marker molecules indicates physical interaction between a first proteins and another molecule, whereby the extent of co-localization or energy-transfer of the two fluorescent reporter proteins indicates the amount of cleaved fluorescent marker and thus the degree of physical interaction.

15. Kit comprising components suitable for carrying out the method as described in claims 13 or 14, comprising at least a fluorescent reporter or fusion protein according to any one of claims 1 to 9, a nucleic acid molecule encoding a fluorescent reporter or fusion protein according to claim 10, and/or a cell comprising a fluorescent reporter or fusion protein or nucleic acid molecule according to claim 11.

**Fig. 1**

Fig. 2

A

B

C

Fig. 2 (cont.)

Fig. 3

**A**

Net1CCG x N$_{ub}$-Cdc14p

GFP-Cdc14p x wt

Fig. 3 (cont.)

## B

○ GFP-Cdc14p x wt
◇ Net1CCG x N$_{ub}$-Cdc14p

Fig. 4

## A

B  Nup49CCG x N_ub-Gsp1p

|  | −30 min | 5 min | 50 min | 100 min | 150 min |

Merge
Cherry
GFP

C  Nup49CCG x N_ub-Gsp1p

□ % GFP RFI
⊖ % Cherry RFI
△ % Conversion

EP 2 772 548 A1

Fig. 4 (cont.)

Fig. 4 (cont.)

**Fig. 4 (cont.)**

Fig. 5

**Fig. 5 (cont.)**

Fig. 5 (cont.)

**Fig. 6**

A Kel1CCG x N$_{ub}$-Spa2p

Merge    Cherry    GFP

Fig. 6 (cont.)

Fig. 6 (cont.)

D

Kel1CCG x N$_{ub}$-Spa2p

Merge    Cherry    GFP

RFI (%)

120
100
80
60
40
20
0

*    **    ***

5 µm

E

Kel1CCG x N$_{ub}$-

Merge    Cherry    GFP

RFI (%)

120
100
80
60
40
20
0

*    **    ***

5 µm

Fig. 7

Fig. 7 (cont.)

**Fig. 7 (cont.)**

**Fig. 8 A**

Fig. 8 B

Stu2CCG x N$_{ub}$-Kar9p

Fig. 8 (cont.)

# EP 2 772 548 A1

Europäisches
Patentamt

European
Patent Office

Office européen
des brevets

# EUROPEAN SEARCH REPORT

Application Number

EP 13 15 6942

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JUDITH MÜLLER ET AL: "Split-Ubiquitin and the Split-Protein Sensors: Chessman for the Endgame", CHEMBIOCHEM, vol. 9, no. 13, 1 September 2008 (2008-09-01), pages 2029-2038, XP055065965, ISSN: 1439-4227, DOI: 10.1002/cbic.200800190 * the whole document * * figure 2 * | 1-15 | INV. C12Q1/02 C12Q1/37 G01N33/50 G01N33/542 G01N33/68 |
| T | DANIEL MORENO ET AL: "A fluorescent reporter for mapping cellular protein-protein interactions in time and space", MOLECULAR SYSTEMS BIOLOGY, vol. 9, 19 March 2013 (2013-03-19), pages 1-13, XP055065967, DOI: 10.1038/msb.2013.3 * the whole document * | 1-15 | |

TECHNICAL FIELDS SEARCHED (IPC)

C12Q
G01N

The present search report has been drawn up for all claims

| Place of search | Date of completion of the search | Examiner |
|---|---|---|
| The Hague | 10 June 2013 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

44

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040170970 A1
- US 5585245 A

**Non-patent literature cited in the description**

- **OZKAYNAK et al.** *EMBO J.,* 1987, vol. 6, 1429
- **AITCHISON, J. D. ; ROUT, M. P.** The yeast nuclear pore complex and transport through it. *Genetics,* 2012, vol. 190, 855-883
- **AL-BASSAM, J. ; VAN BREUGEL, M. ; HARRISON, S. C. ; HYMAN, A.** Stu2p binds tubulin and undergoes an open-to-closed conformational change. *J Cell Biol,* 2006, vol. 172, 1009-1022
- **ALEXANDER, R. P. ; KIM, P. M. ; EMONET, T. ; GERSTEIN, M. B.** Understanding modularity in molecular networks requires dynamics. *Sci Signal,* 2009, vol. 2, 44
- **BACIA, K. ; SCHWILLE, P.** Practical guidelines for dual-color fluorescence cross-correlation spectroscopy. *Nat Protoc,* 2007, vol. 2, 2842-2856
- **BARRAL, Y. ; PARRA, M. ; BIDLINGMAIER, S. ; SNYDER, M.** Nim1-related kinases coordinate cell cycle progression with the organization of the peripheral cytoskeleton in yeast. *Genes Dev,* 1999, vol. 13, 176-187
- **BREITKREUTZ, A. ; CHOI, H. ; SHAROM, J. R. ; BOUCHER, L. ; NEDUVA, V. ; LARSEN, B. ; LIN, Z. Y. ; BREITKREUTZ, B. J. ; STARK, C. ; LIU, G. et al.** A global protein kinase and phosphatase interaction network in yeast. *Science,* 2010, vol. 328, 1043-1046
- **BUCCI, M. ; WENTE, S. R.** In vivo dynamics of nuclear pore complexes in yeast. *J Cell Biol,* 1997, vol. 136, 1185-1199
- **DEEDS, E. J. ; KRIVINE, J. ; FERET, J. ; DANOS, V. ; FONTANA, W.** Combinatorial complexity and compositional drift in protein interaction networks. *PLoS One,* 2012, vol. 7, e32032
- **DÜNKLER, A. ; MULLER, J. ; JOHNSSON, N.** Detecting protein-protein interactions with the split-ubiquitin sensor. *Methods Mol Biol,* 2012, vol. 786, 115-130
- **ECKERT, J. H. ; JOHNSSON, N.** Pex10p links the ubiquitin conjugating enzyme Pex4p to the protein import machinery of the peroxisome. *J Cell Sci,* 2003, vol. 116, 3623-3634
- **GAO, J. T. ; GUIMERA, R. ; LI, H. ; PINTO, I. M. ; SALES-PARDO, M. ; WAI, S. C. ; RUBINSTEIN, B. ; LI, R.** Modular coherence of protein dynamics in yeast cell polarity system. *Proc Natl Acad Sci U S A,* 2011, vol. 108, 7647-7652
- **GAVIN, A. C. ; BOSCHE, M. ; KRAUSE, R. ; GRANDI, P. ; MARZIOCH, M. ; BAUER, A. ; SCHULTZ, J. ; RICK, J. M. ; MICHON, A. M. ; CRUCIAT, C. M. et al.** Functional organization of the yeast proteome by systematic analysis of protein complexes. *Nature,* 2002, vol. 415, 141-147
- **GHAEMMAGHAMI, S. ; HUH, W. K. ; BOWER, K. ; HOWSON, R. W. ; BELLE, A. ; DEPHOURE, N. ; O'SHEA, E. K. ; WEISSMAN, J. S.** Global analysis of protein expression in yeast. *Nature,* 2003, vol. 425, 737-741
- **HRUBY, A. ; ZAPATKA, M. ; HEUCKE, S. ; RIEGER, L. ; WU, Y. ; NUSSBAUMER, U. ; TIMMERMANN, S. ; DUNKLER, A. ; JOHNSSON, N.** A constraint network of interactions: protein-protein interaction analysis of the yeast type II phosphatase Ptc1p and its adaptor protein Nbp2p. *J Cell Sci,* 2011, vol. 124, 35-46
- **JANKE, C. ; MAGIERA, M.M. ; RATHFELDER, N. ; TAXIS, C. ; REBER, S. ; MAEKAWA, H. ; MORENO-BORCHART, A. ; DOENGES, G. ; SCHWOB, E. ; SCHIEBEL, E.** A versatile toolbox for PCR-based tagging of yeast genes: new fluorescent proteins, more markers and promoter substitution cassettes. *Yeast,* 2004, vol. 21, 947-962
- **JOHNSSON, N. ; VARSHAVSKY, A.** Split ubiquitin as a sensor of protein interactions in vivo. *Proc Natl Acad Sci U S A,* 1994, vol. 91, 10340-10344
- **KERPPOLA, T. K.** Design and implementation of bimolecular fluorescence complementation (BiFC) assays for the visualization of protein interactions in living cells. *Nat Protoc,* 2006, vol. 1, 1278-1286
- **KIM, P. M. ; LU, L. J. ; XIA, Y. ; GERSTEIN, M. B.** Relating three-dimensional structures to protein networks provides evolutionary insights. *Science,* 2006, vol. 314, 1938-1941

- **KITAMURA, E. ; TANAKA, K. ; KOMOTO, S. ; KITAMURA, Y. ; ANTONY, C. ; TANAKA, T. U.** Kinetochores generate microtubules with distal plus ends: their roles and limited lifetime in mitosis. *Dev Cell,* 2010, vol. 18, 248-259
- **KROGAN, N. J. ; CAGNEY, G. ; YU, H. ; ZHONG, G. ; GUO, X. ; IGNATCHENKO, A. ; LI, J. ; PU, S. ; DATTA, N. ; TIKUISIS, A. P. et al.** Global landscape of protein complexes in the yeast Saccharomyces cerevisiae. *Nature,* 2006, vol. 440, 637-643
- **LABEDZKA, K. ; TIAN, C. ; NUSSBAUMER, U. ; TIMMERMANN, S. ; WALTHER, P. ; MÜLLER, J. ; JOHNSSON, N.** Sho1p connects the plasma membrane with proteins of the cytokinesis network via multiple isomeric interaction states. *J Cell Sci.,* 2012, vol. 25, 4103-4113
- **LIAKOPOULOS, D. ; KUSCH, J. ; GRAVA, S. ; VOGEL, J. ; BARRAL, Y.** Asymmetric loading of Kar9 onto spindle poles and microtubules ensures proper spindle alignment. *Cell,* 2003, vol. 112, 561-574
- **LASER, H. et al.** *PNAS, Proc Natl Acad Sci U S A.,* 05 December 2000, vol. 97 (25), 13732-7
- **LIPPINCOTT, J. ; LI, R.** Dual function of Cyk2, a cdc15/PSTPIP family protein, in regulating actomyosin ring dynamics and septin distribution. *J Cell Biol,* 1998, vol. 143, 1947-1960
- **LOWDER, M. A. ; APPELBAUM, J. S. ; HOBERT, E. M. ; SCHEPARTZ, A.** Visualizing protein partnerships in living cells and organisms. *Curr Opin Chem Biol,* 2011, vol. 15, 781-788
- **MEITINGER, F. ; BOEHM, M. E. ; HOFMANN, A. ; HUB, B. ; ZENTGRAF, H. ; LEHMANN, W. D. ; PEREIRA, G.** Phosphorylation-dependent regulation of the F-BAR protein Hof1 during cytokinesis. *Genes Dev,* 2011, vol. 25, 875-888
- **MILLER, J. P. ; LO, R. S. ; BEN-HUR, A. ; DESMARAIS, C. ; STAGLJAR, I. ; NOBLE, W. S. ; FIELDS, S.** Large-scale identification of yeast integral membrane protein interactions. *Proc Natl Acad Sci U S A,* 2005, vol. 102, 12123-12128
- **MILLER, R. K. ; CHENG, S. C. ; ROSE, M. D.** Bim1 p/Yeb1 p mediates the Kar9p-dependent cortical attachment of cytoplasmic microtubules. *Mol Biol Cell,* 2000, vol. 11, 2949-2959
- **MORTENSEN, E. M. ; MCDONALD, H. ; YATES, J., 3RD ; KELLOGG, D. R.** Cell cycledependent assembly of a Gin4-septin complex. *Mol Biol Cell,* 2002, vol. 13, 2091-2105
- **MOTULSKY, H. J. ; RANSNAS, L. A.** Fitting curves to data using nonlinear regression: a practical and nonmathematical review. *Faseb J,* 1987, vol. 1, 365-374
- **MÜLLER, J. ; JOHNSSON, N.** Split-ubiquitin and the split-protein sensors: chessman for the endgame. *Chembiochem,* 2008, vol. 9, 2029-2038
- **OHKUNI, K. ; OKUDA, A. ; KIKUCHI, A.** Yeast Nap1-binding protein Nbp2p is required for mitotic growth at high temperatures and for cell wall integrity. *Genetics,* 2003, vol. 165, 517-529
- **OKUZAKI, D. ; NOJIMA, H.** Kcc4 associates with septin proteins of Saccharomyces cerevisiae. *FEBS Lett,* 2001, vol. 489, 197-201
- **PHILIPS, J. ; HERSKOWITZ, I.** Identification of Kel1p, a kelch domain-containing protein involved in cell fusion and morphology in Saccharomyces cerevisiae. *J Cell Biol,* 1998, vol. 143, 375-389
- **PRATT, M. R. ; SCHWARTZ, E. C. ; MUIR, T. W.** Small-molecule-mediated rescue of protein function by an inducible proteolytic shunt. *Proc Natl Acad Sci U S A,* 2007, vol. 104, 11209-11214
- **PRZYTYCKA, T. M. ; SINGH, M. ; SLONIM, D. K.** Toward the dynamic interactome: it's about time. *Brief Bioinform,* 2010, vol. 11, 15-29
- **RABUT, G. ; DOYE, V. ; ELLENBERG, J.** Mapping the dynamic organization of the nuclear pore complex inside single living cells. *Nat Cell Biol,* 2004, vol. 6, 1114-1121
- **SHEU, Y. J. ; SANTOS, B. ; FORTIN, N. ; COSTIGAN, C. ; SNYDER, M.** Spa2p interacts with cell polarity proteins and signaling components involved in yeast cell morphogenesis. *Mol Cell Biol,* 1998, vol. 18, 4053-4069
- **SLAUGHTER, B. D. ; UNRUH, J. R. ; LI, R.** Fluorescence fluctuation spectroscopy and imaging methods for examination of dynamic protein interactions in yeast. *Methods Mol Biol,* 2011, vol. 759, 283-306
- **STRAIGHT, A. F. ; SHOU, W. ; DOWD, G. J. ; TURCK, C. W. ; DESHAIES, R. J. ; JOHNSON, A. D. ; MOAZED, D.** Net1, a Sir2-associated nucleolar protein required for rDNA silencing and nucleolar integrity. *Cell,* 1999, vol. 97, 245-256
- **STYNEN, B. ; TOURNU, H. ; TAVERNIER, J. ; VAN DIJCK, P.** Diversity in genetic in vivo methods for protein-protein interaction studies: from the yeast two-hybrid system to the mammalian split-luciferase system. *Microbiol Mol Biol Rev,* 2012, vol. 76, 331-82
- **TARASSOV, K. ; MESSIER, V. ; LANDRY, C. R. ; RADINOVIC, S. ; SERNA MOLINA, M. M. ; SHAMES, I. ; MALITSKAYA, Y. ; VOGEL, J. ; BUSSEY, H. ; MICHNICK, S. W.** An in vivo map of the yeast protein interactome. *Science,* 2008, vol. 320, 1465-1470
- **UETZ, P. ; GIOT, L. ; CAGNEY, G. ; MANSFIELD, T. A. ; JUDSON, R. S. ; KNIGHT, J. R. ; LOCKSHON, D. ; NARAYAN, V. ; SRINIVASAN, M. ; POCHART, P. et al.** A comprehensive analysis of protein-protein interactions in Saccharomyces cerevisiae. *Nature,* 2000, vol. 403, 623-627

- **USUI, T. ; MAEKAWA, H. ; PEREIRA, G. ; SCHIEBEL, E.** The XMAP215 homologue Stu2 at yeast spindle pole bodies regulates microtubule dynamics and anchorage. *Embo J,* 2003, vol. 22, 4779-4793
- **VIDAL, M. ; CUSICK, M. E. ; BARABASI, A. L.** Interactome networks and human disease. *Cell,* 2011, vol. 144, 986-998
- **VISINTIN, R. ; HWANG, E. S. ; AMON, A.** Cfi1 prevents premature exit from mitosis by anchoring Cdc14 phosphatase in the nucleolus. *Nature,* 1999, vol. 398, 818-823

- **WINEY, M. ; BLOOM, K.** Mitotic spindle form and function. *Genetics,* 2012, vol. 190, 1197-1224
- **WITTKE, S. ; LEWKE, N. ; MULLER, S. ; JOHNSSON, N.** Probing the molecular environment of membrane proteins in vivo. *Mol Biol Cell,* 1999, vol. 10, 2519-2530
- **YU, H. ; BRAUN, P. ; YILDIRIM, M. A. ; LEMMENS, I. ; VENKATESAN, K. ; SAHALIE, J. ; HIRO-ZANE-KISHIKAWA, T. ; GEBREAB, F. ; LI, N. ; SIMONIS, N. et al.** High-quality binary protein interaction map of the yeast interactome network. *Science,* 2008, vol. 322, 104-110